# EUROPEAN PATENT APPLICATION

(11) **EP 4 101 858 A1**
(43) Date of publication of application: **14.12.2022**
(21) Application number: 21178374.1
(22) Date of filing: 08.06.2021
(51) Int. Cl.: C07H 17/08, A01N 43/22

(54) **NOVEL SPINOSYN COMPOUNDS**

(71) Applicant: Acies Bio d.o.o., 1000 Ljubljana (SI)
(72) Inventor: Krajnc, Nika Lendero, 3310 Zalec (SI); Kosec, Gregor, 1000 Ljubljana (SI); Kralj, Petra, 8274 Raka (SI); Fujs, Stefan, 1000 Ljubljana (SI); Svagelj, Mirjan, 0000 Murska Sobota (SI); Camp, Nicholas, London EC4A 3TW (GB)
(74) Representative: Zacco GmbH

(57) **Abstract**

The present invention generally relates to the field of pesticides. More specifically, the present invention relates to novel spinosyn compounds having advantageous properties, processes for their preparation, and their use as pesticides.

## Description

### Field of the invention

The present invention generally relates to the field of pesticides. More specifically, the present invention relates to novel spinosyn compounds having advantageous properties, processes for their preparation, and their use as pesticides.

### Background of the invention

Spinosyns are a large family of natural compounds produced through fermentation of several species of soil bacteria of the genus *Saccharopolyspora.* Multiple structurally diverse spinosyn analogs are biosynthesized concurrently and can be isolated from fermentation broths of producing organisms, for example Saccharopolyspora spinosa, Saccharopolyspora pogona and Saccharopolyspora ASAGF58. Each isolated analog is given a generic name of a "spinosyn" compound, according to *S*. *spinosa,* the first described producing organism. Spinosyns share a core structure, having a polyketide-derived tetracyclic macrolide core with two saccharide moieties linked through glycosidic bonds. Many of the naturally occurring analogs exhibit potent insecticidal activities against many commercially significant pest species that cause extensive damage to crops and other plants. Some of these analogs also exhibit activity against important external parasites of livestock, companion animals and of humans. Spinosyns have a unique mechanism of action (MOA) involving disruption of nicotinic acetylcholine receptors. When compared with many other insecticides, spinosyns generally show greater selectivity toward target insects and lesser activity against many of their beneficial predators.

Butenyl spinosyns are a subgroup of spinosyns, characterized by the butenyl side chain at the carbon 21 (C21) position of the macrolide core. They are produced by *S*. *pogona* (Hahn et al. (2006); EP1373290) and *Saccharopolyspora* ASAGF58 (Guo et al. (2020)). Several butenyl spinosyns, among them butenyl spinosyn α1 were shown to have comparable or even superior insecticidal activity, compared to spinosyn A, the main component of a commercial product Spinosad, produced by S. spinosa (Lewer et al. (2009)). Possibly, superior activity is caused by a more hydrophobic nature of the C21 butenyl side chain compared to C21 ethyl moiety, present in classical spinosyns, such as spinosyn A.

Numerous semi-synthetic spinosyn derivatives were generated in the past, based either on the most abundant natural compounds spinosyn A and spinosyn D or on less abundant natural factors or on compounds, produced by mutants of the producing organisms, such as Saccharopolyspora spinosa (EP0837870, US10570166, WO2017/040878). Butenyl spinosyns were also subjected to further chemical modification, generating numerous semi-synthetic derivatives (EP 1370566). In one study, the possibilities to derivatize the double bond of the C21 butenyl-side chain were explored (Daeuble et al. (2009)). In addition, derivatives based on the ring-expanded 14-membered lactone and C-8 hydroxyl group were produced. In contrast, many of the other sites within the spinosyn/butenyl spinosyn aglycone had been inert to chemical modification because of their saturated hydrocarbon nature. While some of the semi-synthetic derivatives were found to have interesting properties and structure-activity relationships were studied (Kirst (2010)), butenyl spinosyn or its derivatives have so far not been developed as commercial insecticidal products. In contrast, one semi-synthetic derivative, based on C21-ethyl spinosyns, named spinetoram, was developed as a commercial product (Kirst (2010)).

Spinosyns are known to be poorly soluble in water, which complicates the development of liquid formulations. Mixtures of surfactants/emulsifiers, organic solvents and adjuvants have to be added in order to assure adequate solubility of the active compounds. Therefore, novel spinosyn derivatives with improved solubility are needed to simplify formulation development and reduce the negative environmental impacts of these additives on the environment and production cost. In order to increase spinosyn solubility weakly acidic solutions are prepared or addition salts with tartrate ions are prepared (DeAmicis et al. (1997)), resulting in protonated form of the dimethylamino group on the forosamine sugar. Several quaternary ammonium salts of spinosyns have been reported, however, such modification was shown to change the biological activity of spinosyn compounds towards anti-protozoan, anti-viral and anti-cancer activities (WO 2010150100, Ma et al. (2018)). Therefore, such compounds are expected to be less specific and in general less useful as insecticides for use in agriculture.

With growing concerns over the effects of pesticides on aquatic life and beneficial insects (Ramachanderan and Schaefer (2020)) such as pollinators, and inevitable occurrence of resistance among target pests, there is a need to develop and bring to market novel members of the spinosyn family, particularly of the so far neglected butenyl spinosyn class. Preferably, novel molecules should also offer advantages in terms of formulation development.

Against this background, it is an object of the present invention to provide structurally novel spinosyn compounds having advantageous properties.

### Summary of the invention

The present invention provides novel spinosyn compounds having advantageous properties. More specifically, the present invention provides novel spinosyn compounds showing improved solubility compared to existing spinosyn compounds such as butenyl spinosyn which represents a big advantage in preparation of formulations based on aqueous media. In addition, some of the novel spinosyn compounds of the present invention have also shown improved insecticidal activity compared to existing spinosyn compounds such as butenyl spinosyn, Spinosad and spinetoram.

The present invention provides in a main aspect a compound of general formula (I) 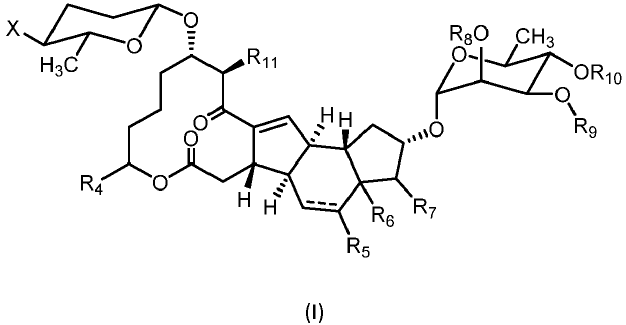 wherein X is NR₂R₃, NHR₂, N⁺R₁R₂R₃ or OR₂ and R₁, R₂, R₃, R₄, R₅, R₆, R₇, R₈, R₉, R₁₀ and R₁₁ are as defined herein;
optionally in the form of a corresponding salt thereof.

The present invention further provides processes for preparation of compounds of the present invention.

The present invention further provides a composition comprising a compound of the present invention and a carrier.

The present invention further provides the use of a compound of the present invention as a pesticide.

The present invention further provides a method for protecting a plant against a plant pest, comprising the step of: applying a compound of the present invention or a composition comprising the same and a carrier to a plant in need thereof.

The present invention can be further summarized by the following items:
1. A compound of general formula (I) 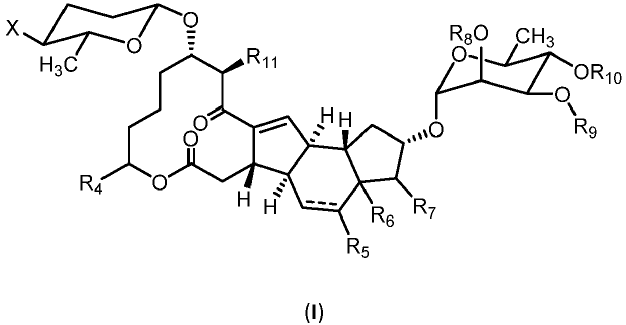 wherein
   the dashed line is a single bond, a double bond or an epoxide;
   X is -NR₂R₃, -NHR₂, -N⁺R₁R₂R₃ or -OR₂;
   R₁ is -(CH₂)n-O-C(O)-R₁b, wherein
      n is an integer ranging from 0 to 20; preferably is an integer ranging from 1 to 10, more preferably is an integer ranging from 1 to 5; and
      Rib is selected from the group consisting of -C(R₂b)₃, -N(R₂b)₂, -OH and -OC(R₂b)₃, wherein
         each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
   R₂ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
   R₃ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
   R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
   R₅ is -H or unsubsituted methyl; preferably is -H;
   R₆ is -OR_{6'} or -OC(O)R_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl;
   R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably R₇ is -H;
   R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
   R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
   R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
   R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl;
   optionally in the form of a corresponding salt thereof.
2. The compound according to item 1, wherein the compound of general formula (I) is a compound of general formula (IIa) 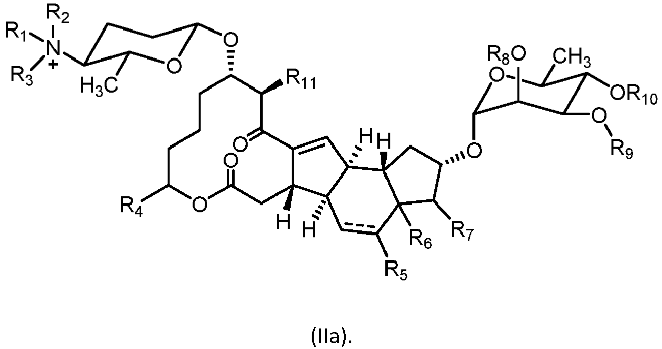
3. The compound according to item 1 or 2, wherein Rib is -C(R₂b)₃.
4. The compound according to item 1 or 2, wherein Rib is -N(R₂b)₂.
5. The compound according to item 1 or 2, wherein Rib is -OC(R₂b)₃.
6. The compound according to item 1 or 2, wherein Rib is -OH.
7. The compound according to The compound according to any one of items 1 to 5, wherein each R₂b is independently selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
8. The compound according to any one of items 1 to 7, wherein n is 1, 2, 3, 4 or 5.
9. The compound according to any one of items 1 to 7, wherein n is 1, 2 or 3.
10. The compound according to any one of items 1 to 7, wherein n is 1.
11. The compound according to item 1, wherein the compound of general formula (I) is a compound of general formula (IIb) 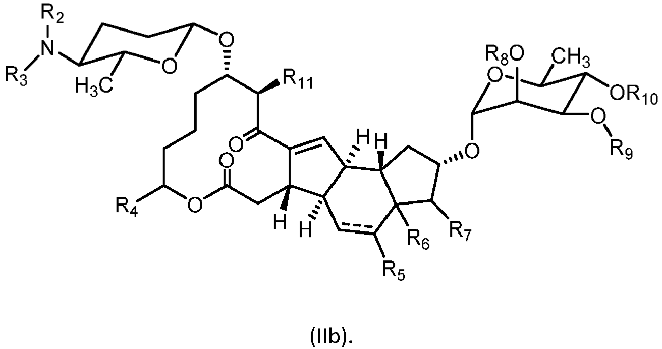
12. The compound according to any one of items 1 to 11, wherein R₃ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, - (C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
13. The compound according to any one of items 1 to 12, wherein R₃ is hydrogen or substituted or unsubsituted C₁-C₆ alkyl.
14. The compound according to any one of items 1 to 13, wherein R₃ is substituted or unsubsituted C₁-C₆ alkyl.
15. The compound according to any one of items 1 to 14, wherein R₃ is unsubsituted C₁-C₆ alkyl.
16. The compound according to any one of items 1 to 15, wherein R₃ is unsubsituted C₁-C₂ alkyl.
17. The compound according to any one of items 1 to 15, wherein R₃ is -CH₃.
18. The compound according to item 1, wherein the compound of general formula (I) is a compound of general formula (IIc) 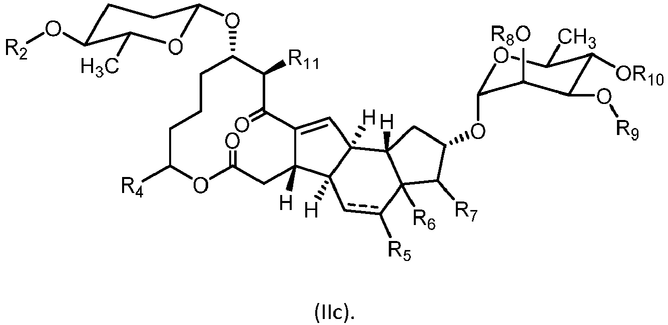
19. The compound according to any one of items 1 to 18, wherein R₂ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted,-(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
20. The compound according to any one of items 1 to 19, wherein R₂ is hydrogen or substituted or unsubsituted C₁-C₆ alkyl.
21. The compound according to any one of items 1 to 19, wherein R₂ is substituted or unsubsituted C₁-C₆ alkyl
22. The compound according to any one of items 1 to 19, wherein R₂ is unsubsituted C₁-C₆ alkyl.
23. The compound according to any one of items 1 to 27, wherein R₂ is -CH₃.
24. The compound according to any one of items 1 to 23, wherein R₁₁ is selected from the group consisting of hydrogen and substituted or unsubsituted methyl.
25. The compound according to item 1, wherein the compound of general formula (I) is a compound of general formula (IIIa) 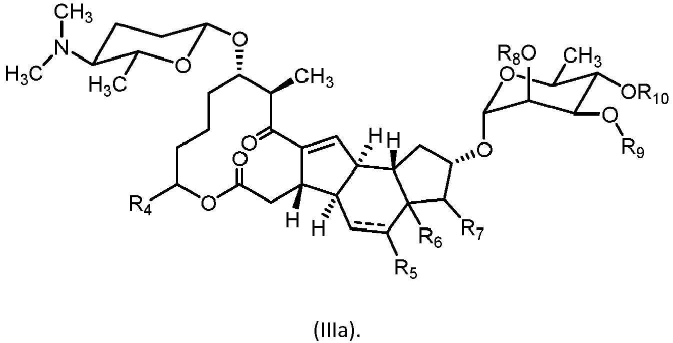
26. The compound according to item 1, wherein the compound of general formula (I) is a compound of general formula (IIIb) 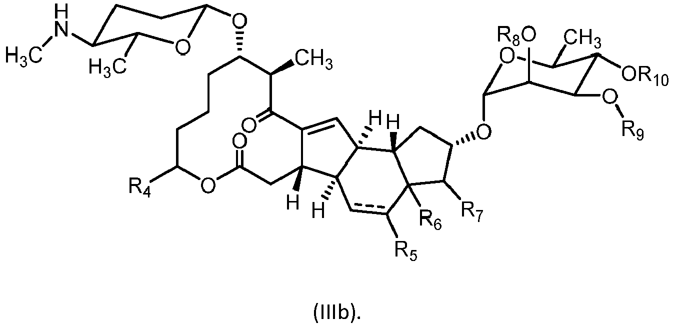
27. The compound according to item 1, wherein the compound of general formula (I) is a compound of general formula (IIIc) 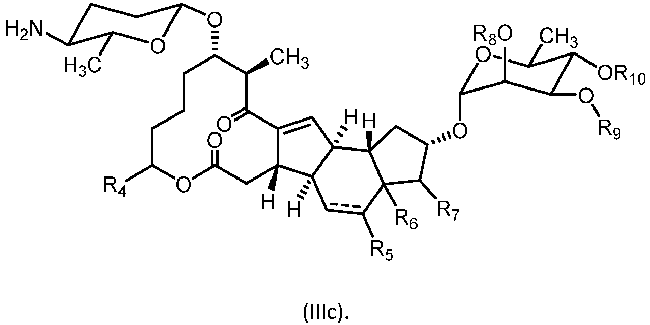
28. The compound according to item 1, wherein the compound of general formula (I) is a compound of general formula (IIId) 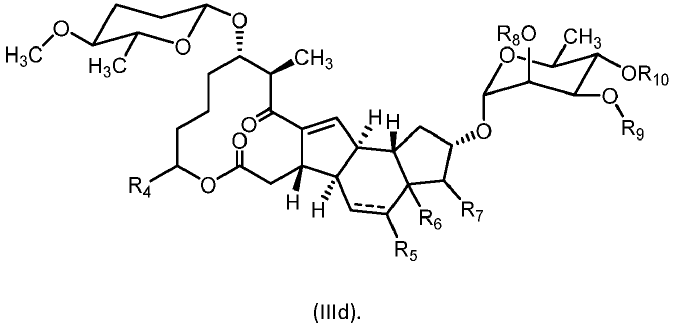
29. The compound according to item 1, wherein the compound of general formula (I) is a compound of general formula (IIIe) 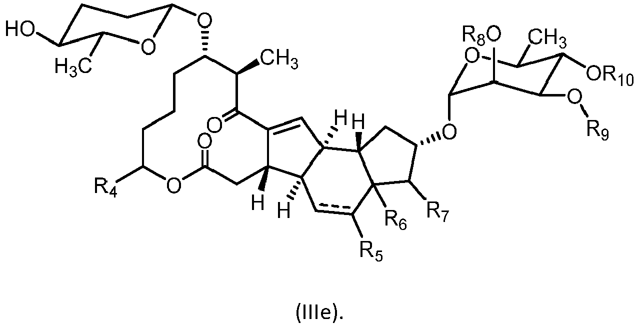
30. The compound according to any one of items 1 to 29, wherein R₄ is unsubstituted ethyl or unsubstituted 1-butenyl.
31. The compound according to any one of items 1 to 30, wherein R₄ is unsubstituted 1-butenyl.
32. The compound according to any one of items 1 to 31, wherein R₅ is -H.
33. The compound according to any one of items 1 to 31, wherein R₅ is -CH₃.
34. The compound according to any one of items 1 to 33, wherein R₆ is -OR_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl.
35. The compound according to any one of items 1 to 33, wherein R₆ is -OC(O)R_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl.
36. The compound according to any one of items 1 to 35, wherein R_{6'} is hydrogen or substituted or unsubsituted C₁-C₆ alkyl.
37. The compound according to any one of items 1 to 36, wherein R_{6'} is hydrogen or substituted or unsubsituted C₁-C₂ alkyl.
38. The compound according to any one of items 1 to 37, wherein R_{6'} is substituted or unsubsituted C₁-C₂ alkyl.
39. The compound according to any one of items 1 to 38, wherein R_{6'} is unsubstituted C₁-C₂ alkyl.
40. The compound according to any one of items 1 to 38, wherein R_{6'} is unsubstituted methyl.
41. The compound according to any one of items 1 to 40, wherein R₇ is -H.
42. The compound according to any one of items 1 to 40, wherein R₇ is -OR_{7'}.
43. The compound according to item 42, wherein R_{7'} is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
44. The compound according to item 42, wherein R_{7'} is substituted or unsubsituted C₁-C₂ alkyl.
45. The compound according to item 42, wherein R_{7'} is unsubsituted methyl.
46. The compound according to any one of items 1 to 45, wherein Rg is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted,-(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
47. The compound according to any one of items 1 to 45, wherein Rg is substituted or unsubsituted C₁-C₆ alkyl.
48. The compound according to any one of items 1 to 45, wherein Rg is substituted or unsubsituted C₁-C₂ alkyl.
49. The compound according to any one of items 1 to 45, wherein Rg is unsubsituted methyl.
50. The compound according to any one of items 1 to 49, wherein R₉ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted,-(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
51. The compound according to any one of items 1 to 49, wherein R₉ is substituted or unsubsituted C₁-C₆ alkyl.
52. The compound according to any one of items 1 to 49, wherein R₉ is substituted or unsubsituted C₁-C₂ alkyl.
53. The compound according to any one of items 1 to 49, wherein R₉ is unsubsituted methyl.
54. The compound according to any one of items 1 to 53, wherein R₁₀ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted,-(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.
55. The compound according to any one of items 1 to 53, wherein R₁₀ is substituted or unsubsituted C₁-C₂ alkyl.
56. The compound according to any one of items 1 to 53, wherein R₁₀ is substituted or unsubsituted C₁-C₂ alkyl.
57. The compound according to any one of items 1 to 53, wherein R₁₀ is unsubsituted methyl.
58. The compound according to any one of items 1 to 57, wherein each of Rg, R₉ and R₁₀ is -CH₃.
59. The compound according to any one of items 1 to 58, wherein the dashed line is a double bond.
60. The compound according to any one of items 1 to 58, wherein the dashed line is a single bond.
61. The compound according to any one of items 1 to 58, wherein the dashed line is an epoxide.
62. The compound according to item 1, which is selected from the group consisting of 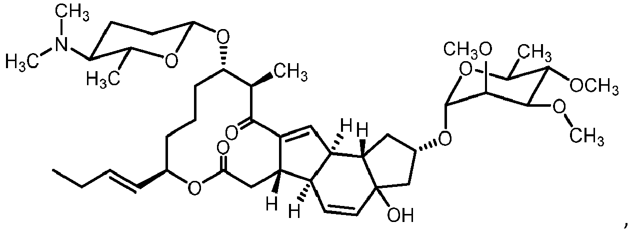 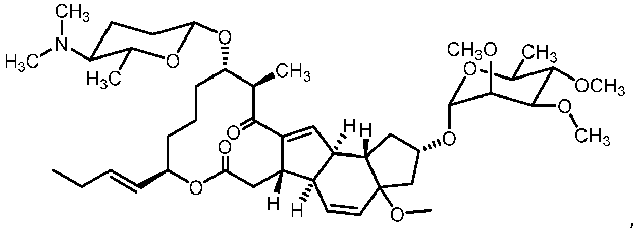 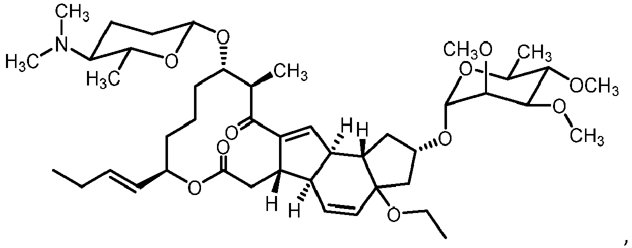 and corresponding salts thereof.
63. Composition comprising a compound according to any one of items 1 to 62, optionally in the form of a corresponding salt thereof, and one or more physiologically acceptable adjuvants.
64. Use of a compound according to any one of items 1 to 62, optionally in the form of a corresponding salt thereof, or a composition according to item 63 as a pesticide.
65. Use of a compound according to any one of items 1 to 62, optionally in the form of a corresponding salt thereof, or a composition according to item 63 as an insecticide.
66. A method for controlling a pest, such as a plant pest, comprises contacting a pest, such as a plant pest, with a compound according to any one of items 1 to 62, optionally in the form of a corresponding salt thereof, or a composition according to item 63.
67. A method for protecting a plant against a plant pest, comprising the step of: applying a compound according to any one of items 1 to 62, optionally in the form of a corresponding salt thereof, or a composition according to item 63 to a plant in need thereof.
68. Use of a compound according to any one of items 1 to 62, optionally in the form of a corresponding salt thereof, or a composition according to item 63 in controlling a pest, such as a plant pest.
69. Use of a compound according to any one of items 1 to 62, optionally in the form of a corresponding salt thereof, or a composition according to item 63 in protecting a plant against a plant pest.
70. The method according to item 66 or 67 or the use according to item 68 or 69, wherein the plant pest is an insect or mite.
71. The method according to item 66 or 67 or the use according to item 68 or 69, wherein the plant pest is an insect.
72. The method according to any one of items 66 to 67 or the use according to item 68 or 69, wherein the plant pest is a herbivorous insect.
73. The method or use according to item 71 or 72, wherein the plant pest is a larva of the insect.
74. The method or use according to item 71 or 72, wherein the plant pest is an imago of the insect.
75. The method or use according to any one of items 66 to 74, wherein the insect is of the order *Coleoptera, Lepidoptera, Diptera, Hemiptera, Orthoptera, Hymenoptera, Thysanoptera* or *Blattodea.*
76. The method or use according to any one of items 66 to 74, wherein the insect is of the family *Aphididae, Aleyrodidoe, Agromizydae, Byturidae, Chrysomelidae, Cicadellidae, Crambidae, Curculionidae, Drosophilidae, Elateridae, Eriophyidae, Pentatomidae, Plutellidae, Gelechiidae, Gracillariidae, Hadeninae, Thripidae, Tortricidae, Nitidulidae, Noctuidoe, Pyralidae, Pieridae, Scarabeidae or Tetranychidae.*
77. The method or *use according to* any one of items 66 to 74, wherein the insect is of the genus *Aphis, Agriotes, Anarsia, Bermisia, Byturus, Bactrocera, Crioceris, Chilo, Cydia, Cnaphalocrocis, Chrysodeixis, Ceratitis, Dacus, Diabrotica, Diatraea, Drosophila, Euschistus, Eupoecillia, Frankliniella, Halyomorpha, Helicoverpa, Heliothis, Heliothrips, Hercinothrips, Leptinotarsa, Lilioceris, Limothrips, Lobesia, Myzus, Melolontha, Nilaparvata, Ostrinia, Phyllotreto, Phyllocnistis, Popillia, Plutella, Pieris, Sesamia, Spodoptera, Thrips, Tribolium, Trialeurodes, Trichoplusia, Tortrix,Tetranychus, Tuta, Grapholita, Clysia, Popillia, Anthonomus, Meligethes, Rhopalosiphum, Macrosiphum, Acrosternum, Nezara or Piezodorus.*
78. The method or use according to any one of items 66 to 74, wherein the insect is *Aphis gossypii, Anarsia lineatella, Agriotes spp., Agriotes lineatus, Agriotes obscurus, Agriotes ustulatus, Agriotes sputator, Bemisia tabaci, Byturus tomentosus, Bactrocera carambolae, Bactrocera invadens, Bactrocera cucurbitae, Bactrocera dorsalis complex, Chilo suppressalis, Cydia pomonella, Crioceris duodecimpunctata, Ceratitis capitate, Ceratitis rosa, Ceratitis cosyra, Ceratitis fasciventris, Chrysodeixis includens, Cydia pomonella, Diabrotica virgifera virgifera, Dacus frontalis, Dacus bivittatus, Dacus vertebratus, Dacus ciliates, Diatraea venostata, Drosophila suzukii, Eupoecillia ambliguella, Frankliniella occidentalis, Halyomorpha halys, Helicoverpa zea, Helicoverpa armigera, Hercinothrips bicinctus, Hercinothrips femoralis, Leptinotarsa decemlineata, Lilioceris merdigera, Lilioceris lilii, Limothrips cerealium, Lobesia botrana, Melolontha melolontha, Myzus persicae, Nilaparvata lugens, Ostrinia nubilialis, Pononychus ulmi, Phyllotreta spp., Phyllotreta cruciferae, Phyllotreta striolata, Popillia japonica, Phyllocnistis citrella, Plutella xylostella, Pieris brassicae, Peridroma saucia, Sesamia Sesamia, Spodoptera litura, Spodoptera frugiperda, Thrips simplex, Thrips palmi, Thrips tabaci, Tribolium castaneum, Trialeurodes vaporariorum, Tuta absoluta, Tetranychus urticae, Sesamia nonagrioïdes, Sopdoptera exigua, Spodoptera eridania, Trichoplusia ni, Heliothis virescens; Grapholita molesta, Clysia ambiguella, Popillia japonica, Anthonomus grandis, Meligethes aeneus, Ceutorhynchus napi, Psylliodes chrysocephala, Sitobion avenae, Rhopalosiphum padi, Macrosiphum euphorbiae, Acrosternum hilare, Nezara viridula or Piezodorus guildinii.*
79. The method according to any one of items 67 and 70 to 78 or the use according to any one of items 66 to 75, wherein the plant is a crop plant.
80. Use of a compound according to any one of items 1 to 62, optionally in the form of a corresponding salt thereof, or a composition according to item 63 in agriculture.
81. Process for the preparation of a compound of general formula (I) as defined in any one of items 1 to 62 (with R₆ being -H or -OR_{6'}), said process comprising reacting a compound of formula (p-I) 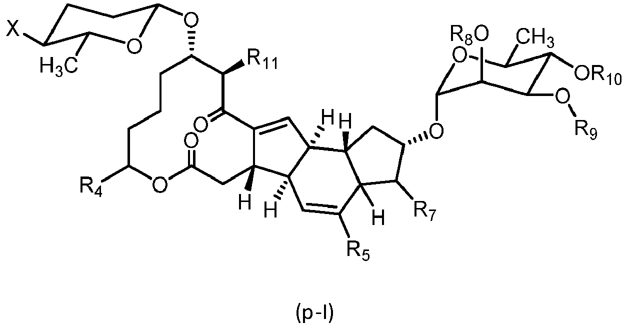 with SeO₂ to obtain a compound according to general formula (I-xi) 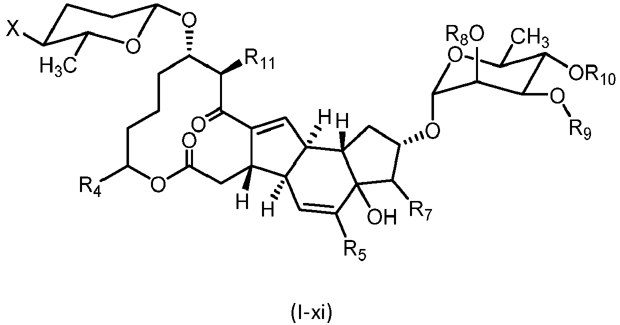 optionally further reacting the resulting compound of general formula (I-x) with a compound of a general formula R_{6'}-Z to obtain a compound of general formula (I-xii) 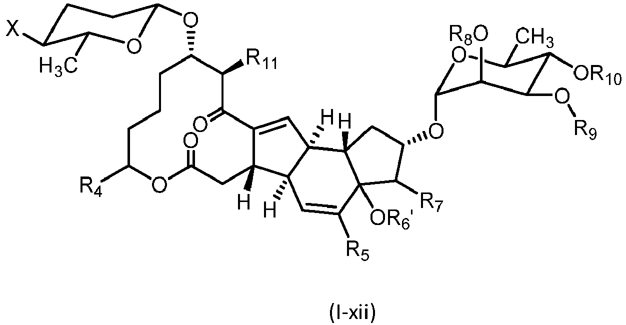 optionally further reacting the compound of general formula (I-xii) with hydrogen (H₂);
   wherein Z is a halogen, preferably CI, Br or I, R_{6'} is substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and-(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl; and X, R₄, R₅, R₇, R₈, R₉, R₁₀ and R₁₁ are as defined above.
82. Process for the preparation of a compound of general formula (I) as defined in any one of items 1 to 61 (with R₆ being -OC(O)H), said process comprising reacting a compound of formula (p-I) 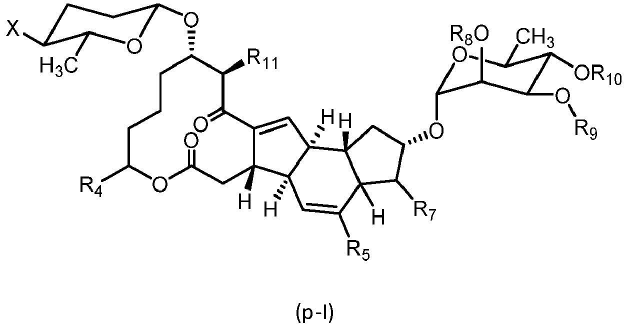 with SeO₂ in the presence of formic acid to obtain a compound according to general formula (I-xxi) 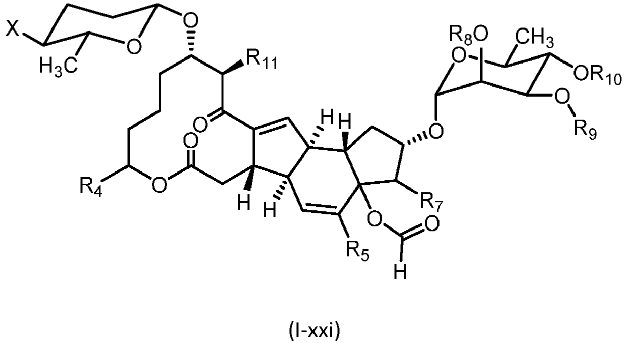 optionally further reacting the compound of general formula (I-xxi) with hydrogen (H₂); wherein X, R₄, R₅, R₇, R₈, R₉, R₁₀ and R₁₁ are as defined above.
83. Process for the preparation of a compound of general formula (I) as defined in any one of items 1 to 61 (with R₆ being -OC(O)R_{6'}), said process comprising reacting a compound of formula (p-I) 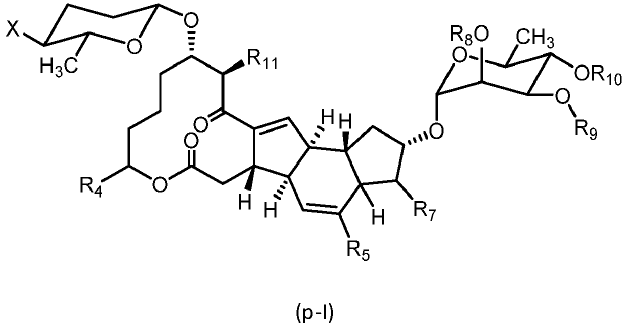 with SeO₂ to obtain a compound according to general formula (I-xi) 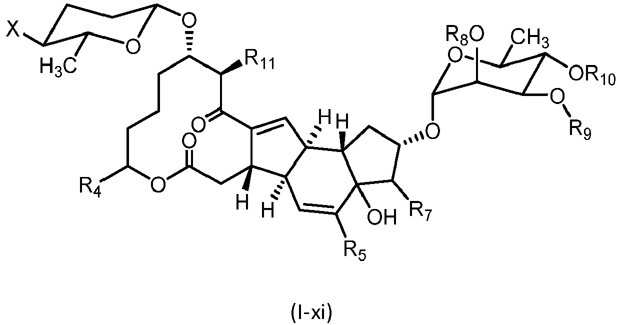 further reacting the resulting compound of general formula (I-xi) with a compound of a general formula R_{6'}C(O)-Z or a compound of a general formula R_{6'}C(O)-O-(O)C- R_{6'} to obtain a compound of general formula (I-xxxi) 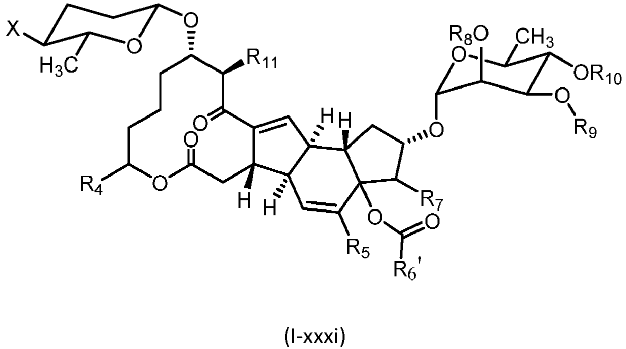 optionally further reacting the compound of general formula (I-xxxi) with hydrogen (H₂); wherein Z is a halogen, preferably CI, Br or I, R_{6'} is substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and - (C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl; and X, R₄, R₅, R₇, R₈, R₉, R₁₀ and R₁₁ are as defined above.

The present invention is now described in more detail.

### Detailed description of the invention

The present invention provides novel spinosyn compounds having advantageous properties. More specifically, the present invention provides novel spinosyn compounds showing improved solubility compared to existing spinosyn compounds such as butenyl spinosyn which represents a big advantage in preparation of formulations based on aqueous media. In addition, some of the novel spinosyn compounds of the present invention have also shown improved insecticidal activity compared to existing spinosyn compounds such as butenyl spinosyn, Spinosad and spinetoram.

In one aspect, the present invention provides a compound of general formula (I) 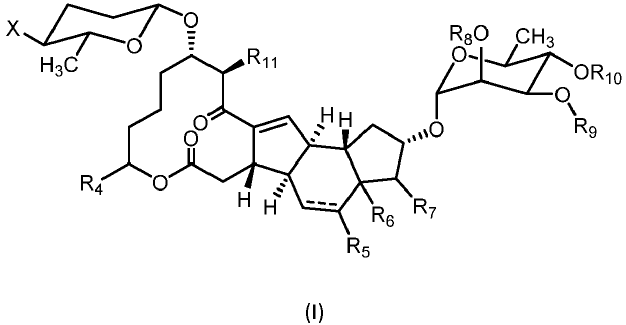 wherein
the dashed line is a single bond, a double bond or an epoxide;
X is -NR₂R₃, -NHR₂, -N⁺R₁R₂R₃ or -OR₂;
R₁ is -(CH₂)n-O-C(O)-R₁b, wherein
   n is an integer ranging from 0 to 20; preferably is an integer ranging from 1 to 10, more preferably is an integer ranging from 1 to 5; and
   Rib is selected from the group consisting of -C(R₂b)₃, -N(R₂b)₂, -OH and -OC(R₂b)₃, wherein
      each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₂ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
R₃ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -OR_{6'} or -OC(O)R_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl;
R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably R₇ is -H;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl;
optionally in the form of a corresponding salt thereof.

According to some embodiments, X is -NR₂R₃.

According to some embodiments, X is -NHR₂.

According to some embodiments, X is -WR₁R₂R₃.

According to some embodiments, X is -OR₂.

According to some embodiments, the compound of general formula (I) is a compound of general formula (IIa) 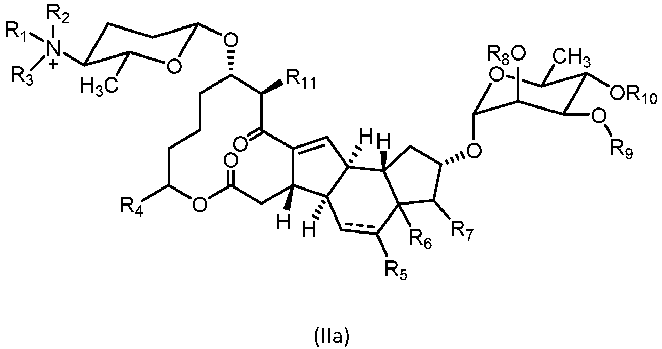 wherein the dashed line, R₁, R₂, R₃, R₄, R₅, R₆, R₇, R₈, R₉, R₁₀ and R₁₁ are as defined herein;
optionally in the form of a corresponding salt thereof.

According to some embodiments, Rib is -C(R₂b)₃.

According to some embodiments, Rib is -N(R₂b)₂.

According to some embodiments, Rib is -OH.

According to some embodiments, Rib is -OC(R₂b)₃.

According to some embodiments, each R₂b is independently selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, each R₂b is independently selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, each R₂b is independently selected from the group consisting of hydrogen and substituted or unsubsituted C₁-C₆ alkyl.

According to some embodiments, each R₂b is independently selected from the group consisting of hydrogen and unsubsituted C₁-C₆ alkyl.

According to some embodiments, each R₂b is independently selected from the group consisting of hydrogen and unsubsituted C₁-C₄ alkyl.

According to some embodiments, each R₂b is independently selected from the group consisting of hydrogen and -CH₃.

According to some embodiments, n is an integer ranging from 1 to 10.

According to some embodiments, n is an integer ranging from 1 to 5.

According to some embodiments, n is 1, 2, 3 or 4.

According to some embodiments, n is 1, 2 or 3.

According to some embodiments, n is 1 or 2.

According to some embodiments, n is 1.

According to some embodiments, n is 2.

According to some embodiments, n is 3.

According to some embodiments, n is 4.

According to some embodiments, n is 5.

According to some embodiments, the compound of general formula (I) is a compound of general formula (IIb) 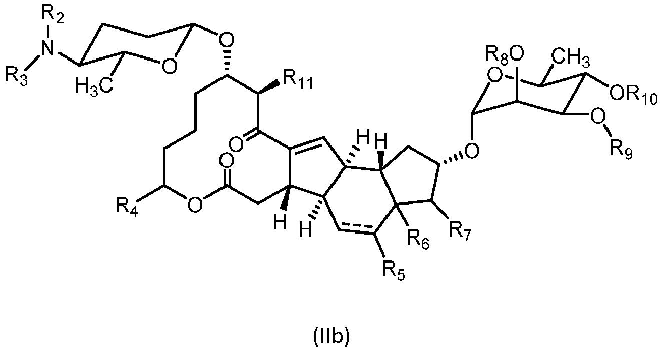 wherein the dashed line, R₂, R₃, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIc) 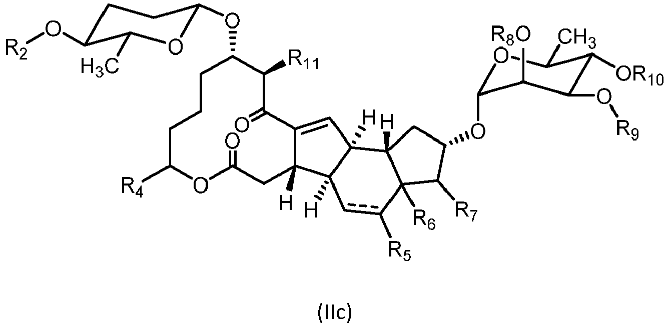 wherein the dashed line, R₂, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIIa) 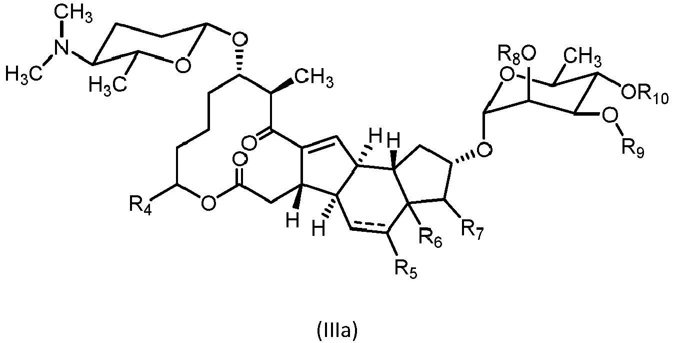 wherein the dashed line, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIIb) 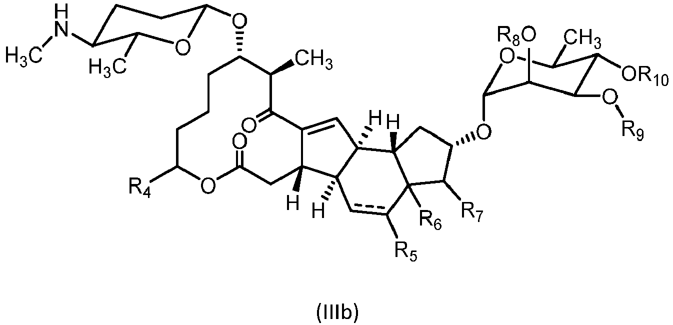 wherein the dashed line, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIIc) 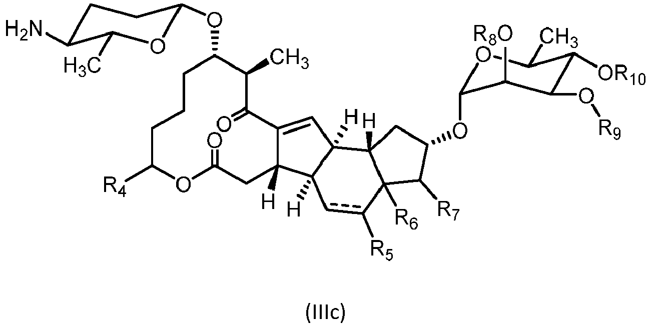 wherein the dashed line, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIId) 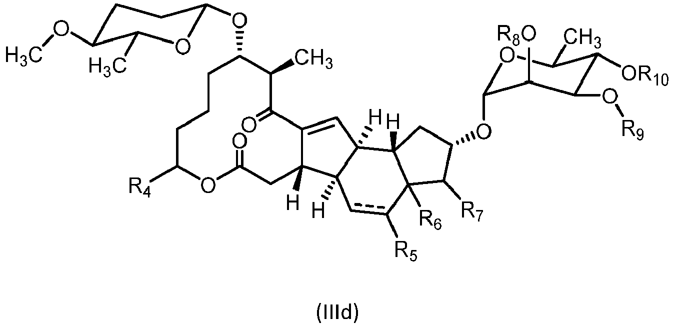 wherein the dashed line, R₄, R₅, R₆, R₇, R₈, R₉, R₁₀ and R₁₁ are as defined herein;
optionally in the form of a corresponding salt thereof.

According to some embodiments, the compound of general formula (I) is a compound of general formula (IIIe) 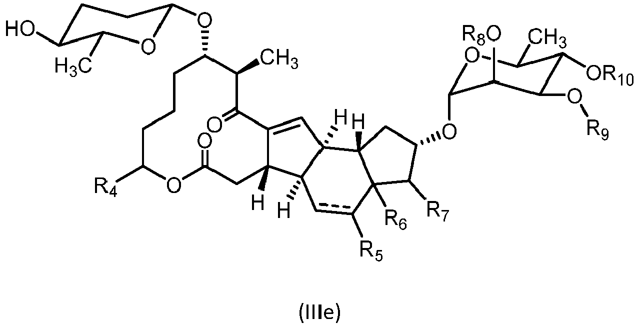 wherein the dashed line, R₄, R₅, R₆, R₇, R₈, R₉, R₁₀ and R₁₁ are as defined herein;
optionally in the form of a corresponding salt thereof.

According to some embodiments, R₂ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₂ is hydrogen or substituted or unsubsituted C₁-C₆ alkyl.

According to some embodiments, R₂ is substituted or unsubsituted C₁-C₆ alkyl.

According to some embodiments, R₂ is unsubsituted C₁-C₆ alkyl.

According to some embodiments, R₂ is unsubsituted C₁-C₂ alkyl.

According to some embodiments, R₂ is -CH₃.

According to some embodiments, R₃ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₃ is hydrogen or substituted or unsubsituted C₁-C₆ alkyl.

According to some embodiments, R₃ is substituted or unsubsituted C₁-C₆ alkyl.

According to some embodiments, R₃ is unsubsituted C₁-C₆ alkyl.

According to some embodiments, R₃ is unsubsituted C₁-C₂ alkyl.

According to some embodiments, R₃ is -CH₃.

According to some embodiments, R₄ is unsubstituted ethyl or unsubstituted 1-butenyl.

According to some embodiments, R₄ is unsubstituted 1-butenyl.

According to some embodiments, R₅ is -H.

According to some embodiments, R₅ is -CH₃.

According to some embodiments, R₆ is -OR_{6'}.

According to some embodiments, R₆ is -OC(O)R_{6'}.

According to some embodiments, R_{6'} is hydrogen or substituted or unsubsituted C₁-C₆ alkyl.

According to some embodiments, R_{6'} is hydrogen or substituted or unsubsituted C₁-C₂ alkyl.

According to some embodiments, R_{6'} is substituted or unsubsituted C₁-C₂ alkyl.

According to some embodiments, R_{6'} is unsubstituted C₁-C₂ alkyl.

According to some embodiments, R_{6'} is unsubstituted methyl.

According to some embodiments, R₇ is -H.

According to some embodiments, R₇ is OR_{7'}.

According to some embodiments, R_{7'} is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R_{7'} is selected from the group consisting of hydrogen, substituted or unsubsituted C₁-C₆ alkyl, substituted or unsubsituted C₂-C₆ alkenyl, and substituted or unsubsituted C₂-C₆ alkynyl.

According to some embodiments, R_{7'} is selected from the group consisting of hydrogen and substituted or unsubsituted C₁-C₆ alkyl.

According to some embodiments, R_{7'} is substituted or unsubsituted C₁-C₂ alkyl; preferably is -CH₃.

According to some embodiments, Rg is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, Rg is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, Rg is hydrogen or substituted or unsubsituted C₁-C₆ alkyl.

According to some embodiments, Rg is substituted or unsubsituted C₁-C₂ alkyl; preferably is -CH₃.

According to some embodiments, R₉ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₉ is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₉ is hydrogen or substituted or unsubsituted C₁-C₆ alkyl.

According to some embodiments, R₉ is substituted or unsubsituted C₁-C₂ alkyl; preferably is -CH₃.

According to some embodiments, R₁₀ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₁₀ II) is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₁₀ is hydrogen or substituted or unsubsituted C₁-C₆ alkyl.

According to some embodiments, R₁₀ is substituted or unsubsituted C₁-C₂ alkyl; preferably is -CH₃.

According to some embodiments, each of Rg, R₉ and R₁₀ is substituted or unsubsituted C₁-C₆ alkyl.

According to some embodiments, each of Rg, R₉ and R₁₀ is unsubsituted C₁-C₆ alkyl.

According to some embodiments, each of Rg, R₉ and R₁₀ is -CH₃.

According to some embodiments, R₁₁ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₁₁ is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₁₁ is hydrogen or substituted or unsubsituted methyl.

According to some embodiments, R₁₁ is hydrogen.

According to some embodiments, R₁₁ is substituted or unsubsituted methyl.

According to some embodiments, R₁₁ is -CH₃.

According to some embodiments, the dashed line is a double bond.

According to some embodiments, the dashed line is a single bond.

According to some embodiments, the dashed line is an epoxide.

According to some embodiments, the compound is selected from the group consisting of 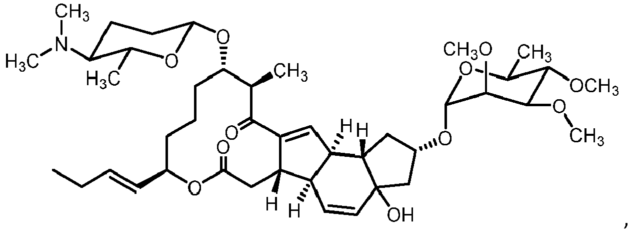 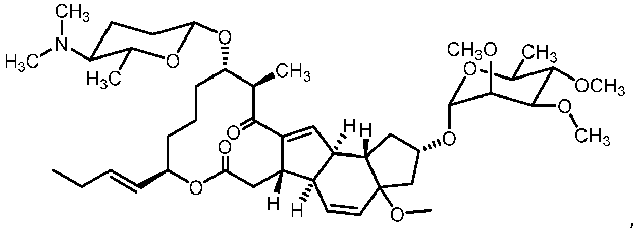 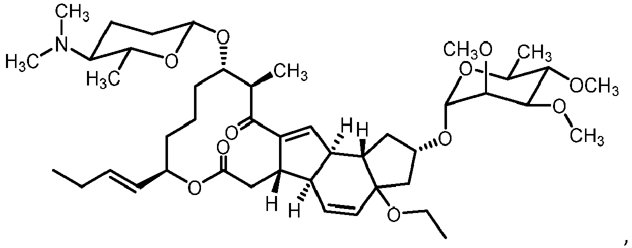 and corresponding salts thereof.

According to some embodiments, the compound is selected from the group consisting of 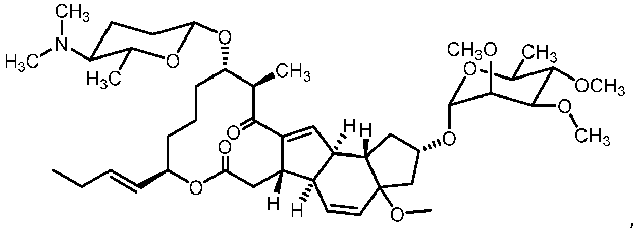 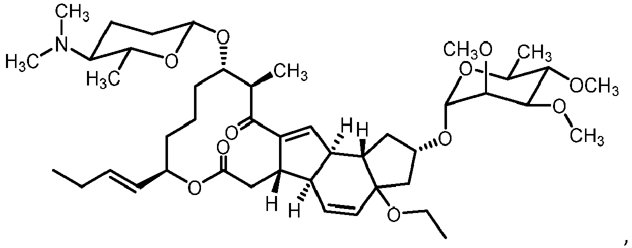 and corresponding salts thereof.

According to some embodiments, the compound is 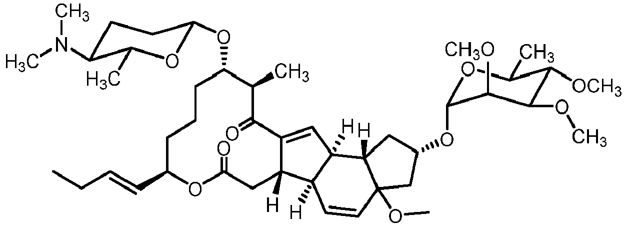 or a corresponding salt thereof.

The present invention further provides a composition comprising a compound according the present invention, optionally in the form of a corresponding salt thereof, and one or more physiologically acceptable adjuvants.

Composition of the present invention include concentrated versions, in which the present active agent is present in a concentration of from 0.001 to 98.0 percent, with the remaining content being physiologically acceptable carriers. Such compositions, especially those with less than 50 percent of the present compound, can sometimes be used directly, but these compositions can also be diluted with other physiologically acceptable carriers to form more dilute treating formulations. These latter compositions can include the active agent in lesser concentrations of from 0.001 to 0.1 percent. Compositions are prepared according to the procedures and formulas which are conventional in the agricultural or pest control art. The compositions may be concentrated and dispersed in water or may be used in the form of a dust, bait or granular formulation. The dispersions are typically aqueous suspensions or emulsions prepared from concentrated formulations of the compounds. The watersoluble or water-suspension or emulsifiable formulations are either solids, wettable powders, or liquids, known as emulsifiable concentrates or aqueous suspensions. Wettable powders may be agglomerated or compacted to form water dispersible granules. These granules comprise mixtures of compound, inert carriers and surfactants. The concentration of the compound is typically between about 0 .1% to about 90% by weight. The inert carrier is typically attapulgite clays, montmorillonite clays and the diatomaceous earths or purified silicates. Surfactants comprise typically about 0.5% to about 10% of the wettable powder. Surfactants include sulfonated lignins, condensed napthalenesulfonates, the napthalenesulfonates, alkyl-benenesulfonates, alkysulfonates or nonionic surfactants such as ethylene oxide adducts of alkylphenols or mixtures thereof. Emulsifiable concentrates of the derivatives of the invention typically range from about 50 to about 500 grams of spinosyn derivative per liter of liquid, equivalent to about 10% to about 50%, dissolved in an inert carrier which is a mixture of a water immiscible solvent and emulsifiers. Organic solvents include organics such as xylenes, and petroleum fractions such as high-boiling naphthlenic and olefinic portions of petroleum which include heavy and aromatic naphtha. Other organics may also be used such as terpenic solvents -rosin derivatives, aliphatic ketones such as cyclohexanone and complex alcohols. Emulsifiers for emulsifiable concentrates are typically mixed ionic and/or nonionic surfactants such as those mentioned herein or their equivalents. Aqueous suspensions may be prepared containing water-insoluble spinosyn derivatives, where the compounds are dispersed in an aqueous vehicle at a concentration typically in the range of between about 5% to about 50% by weight. The suspensions are prepared by finely grinding the compound and vigorously mixing it into a vehicle of water, surfactants, and dispersants. Inert ingredients such as inorganic salts and synthetic or natural gums may also be employed to increase the density and/or viscosity of the aqueous vehicle as is desired. Precipitated flowables may be prepared by dissolving at least one spinosyn derivative of the invention in a water-miscible solvent and surfactants or surface active polymers. When these formulations are mixed with water, the active spinosyn derivative precipitates with the surfactant controlling the size of the resulting micro-crystalline precipitate. The size of the crystal can be controlled through the selection of specific polymer and surfactant mixtures. The spinosyn derivatives may also be applied as a granular composition that is applied to the soil. The granular composition typically contains from about 0.5% to about 10% by weight of the derivative. The spinosyn derivative is dispersed in an inert carrier which is typically clay or an equivalent substance. Generally, granular compositions are prepared by dissolving the compounds of the invention in a suitable solvent and applying it to a granular carrier which has been pre-formed to the desirable particle size. The particle size is typically between about 0.5 mm to 3 mm. The granular compositions may also be prepared by forming a dough or paste of the carrier and compound, drying the combined mixture, and crushing the dough or paste to the desired particle size.

The spinosyn compounds may also be combined with an appropriate organic solvent. The organic solvent is typically a bland petroleum oil that is widely used in the agricultural industry. These combinations are typically used as a spray. More typically, the spinosyn compounds are applied as a dispersion in a liquid carrier, where the liquid carrier is water. The compounds may also be applied in the form of an aerosol composition. The compound is dissolved in an inert carrier, which is a pressure-generating propellant mixture. The aerosol composition is packaged in a container, where the mixture is dispersed through an atomizing valve. Propellant mixtures contain either low-boiling halocarbons, which may be mixed with organic solvents or aqueous suspensions pressurized with inert gases or gaseous hydrocarbons. The compounds may be applied to any locus inhabited by an insect or mite. Such locus typically is cotton, soybean and vegetable crops, fruit and nut trees, grape vines, houses and ornamental plants. The amount of the spinosyn compound applied to the loci of insects and mites can be determined by those skilled in the art. Generally, the concentrations of from about 10 ppm to about 5,000 ppm provide the desired control. For crops such as soybeans and cotton, the rate of application is about 0.01 to about 1 kg/ha, where the spinosyn derivative is applied in a 5 to 50 gal/A spray formulation.

The composition can be formulated in a liquid concentrate, ready-to-use (RTU) liquid spray, dust, or solid form. The formulation chosen will depend on the use of the product. The following general treatment methods are preferably suitable for carrying out the seed treatment, or plant propagation material treatment, according to the invention: dry treatments (preferably with addition of adhesion promoters such as, for example, liquid paraffin or talc), and, if appropriate, colorants, slurry treatments (preferably with addition of wetters, dispersants, emulsifiers, adhesives, inert fillers and colorants), aqueous liquid treatments (preferably with addition of emulsifiers, dispersants, thickeners, antifreeze agents, polymers, adhesives and colorants), solvent-based liquid treatments (with addition of solvents and colorants), emulsion treatments (with addition of emulsifiers, solvents and colorants).

The present invention further provides the use of a compound, optionally in the form of a corresponding salt thereof, or composition according to the present invention as a pesticide.

The present invention further provides the use of a compound according to the present invention, optionally in the form of a corresponding salt thereof, or a composition according to the present invention as an insecticide.

The present invention further provides the use of a compound, optionally in the form of a corresponding salt thereof, or composition according to the present invention in controlling a pest, such as a plant pest.

The present invention further provides the use of a compound, optionally in the form of a corresponding salt thereof, or composition according to the present invention in protecting a plant against a plant pest.

The present invention further provides a method for controlling a pest, such as a plant pest, comprises contacting a pest, such as a plant pest, with a compound, optionally in the form of a corresponding salt thereof, or composition of the present invention.

The present invention further provides a method for protecting a plant against a plant pest, comprising the step of: applying a compound, optionally in the form of a corresponding salt thereof, or composition of the present invention to a plant in need thereof.

The compound or composition as defined above may be used on any plant in need of being protected against a plant pest. The plant may be an angiosperm or gymnosperm. According to some embodiments, the plant is an angiosperm. According to some embodiment, the plant is a gymnosperm. The plant may be a dicot or monocot. According to some embodiments, the plant is a dicot. According to some embodiment, the plant is a monocot. The plant may be a food plant (i.e. a plant some parts of which provides food for animal or human consumption), such as fruit plant.

The plant may be a crop plant, such as a food crop plant. According to certain embodiments, the plant is a food crop plant selected from the group consisting of pepper plant, cocoa plant, tomato plant, potato plant, maize plant, wheat plant and rice plant. The plant may be a tobacco plant, such as *Nicotiana tabacum.*

The plant pest can be an insect, such as a herbivorous insect, an arachnid or a nematode. Therefore, according to certain embodiments, the plant pest is an insect.

According to some embodiments, the plant pest is a herbivorous insect.

According to some embodiments, the insect is of the order *Coleoptera, Lepidoptera, Diptera, Hemiptera, Orthoptera, Hymenoptera, Thysanoptera* or *Blattodea.*

According to some embodiments, the insect is of the order *Coleoptera* or *Lepidoptera.*

According to some embodiments, the insect is of the order *Coleoptera.*

According to some embodiments, the insect is of the order *Lepidoptera.*

According to some embodiments, the insect is of the family *Aphididae, Aleyrodidae, Agromizydae, Byturidae, Chrysomelidae, Cicadellidae, Crambidae, Curculionidae, Drosophilidae, Elateridae, Eriophyidae, Pentatomidae, Plutellidae, Gelechiidae, Gracillariidae, Hadeninae, Thripidae, Tortricidae, Nitidulidae, Noctuidae, Pyralidae, Pieridae, Scarabeidae or Tetranychidae.*

According to some embodiments, the insect is of the genus *Aphis, Agriotes, Anarsia, Bermisia, Byturus, Bactrocera, Crioceris, Chilo, Cydia, Cnaphalocrocis, Chrysodeixis, Ceratitis, Dacus, Diabrotica, Diatraea, Drosophila, Euschistus, Eupoecillia, Frankliniella, Halyomorpha, Helicoverpa, Heliothis, Heliothrips, Hercinothrips, Leptinotarsa, Lilioceris, Limothrips, Lobesia, Myzus, Melolontha, Nilaparvata, Ostrinia, Phyllotreta, Phyllocnistis, Popillia, Plutella, Pieris, Sesamia, Spodoptera, Thrips, Tribolium, Trialeurodes, Trichoplusia, Tortrix, Tetronychus, Tuta, Grapholita, Clysia, Popillia, Anthonomus, Meligethes, Rhopalosiphum, Macrosiphum, Acrosternum, Nezara or Piezodorus.*

According to some embodiments, the insect is *Aphis gossypii, Anarsia lineatella, Agriotes spp., Agriotes lineatus, Agriotes obscurus, Agriotes ustulatus, Agriotes sputator, Bemisia tabaci, Byturus tomentosus, Bactrocera carambolae, Bactrocera invadens, Bactrocera cucurbitae, Bactrocera dorsalis complex, Chilo suppressalis, Cydia pomonella, Crioceris duodecimpunctata, Ceratitis capitate, Ceratitis rosa, Ceratitis cosyra, Ceratitis fasciventris, Chrysodeixis includens, Cydia pomonella, Diabrotica virgifera virgifera, Dacus frontalis, Dacus bivittatus, Dacus vertebratus, Dacus ciliates, Diatraea venostata, Drosophila suzukii, Eupoecillia ambliguella, Frankliniella occidentalis, Halyomorpha halys, Helicoverpa zea, Helicoverpa armigera, Hercinothrips bicinctus, Hercinothrips femoralis, Leptinotarsa decemlineata, Lilioceris merdigera, Lilioceris lilii, Limothrips cerealium, Lobesia botrana, Melolontha melolontha, Myzus persicae, Nilaparvata lugens, Ostrinia nubilialis, Panonychus ulmi, Phyllotreta spp., Phyllotreta cruciferae, Phyllotreta striolata, Popillia japonica, Phyllocnistis citrella, Plutella xylostella, Pieris brassicae, Peridroma saucia, Sesamia Sesamia, Spodoptera litura, Spodoptera frugiperda, Thrips simplex, Thrips palmi, Thrips tabaci, Tribolium castaneum, Trialeurodes vaporariorum, Tuta absoluta, Tetranychus urticae, Sesamia nonagrioïdes, Sopdoptera exigua, Spodoptera eridania, Trichoplusia ni, Heliothis virescens; Grapholita molesta, Clysia ambiguella, Popillia japonica, Anthonomus grandis, Meligethes aeneus, Ceutorhynchus napi, Psylliodes chrysocephala, Sitobion avenae, Rhopalosiphum padi, Macrosiphum euphorbiae, Acrosternum hilare, Nezara viridula or Piezodorus guildinii.*

The plant pest may be a larva or an imago of the insect. According to some embodiments, the plant pest is a larva of the insect. The larva may be in any stage of larval development, such as a larval stage selected from the group consisting of L1, L2, L3, L4, and L5. According to some embodiments, the plant pest is an imago of the insect.

The present invention further provides the use of a compound, optionally in the form of a corresponding salt thereof, or composition according to the present invention in agriculture.

Generally, the compound or composition can be applied to a plant in need thereof in any suitable dose, frequency and method of administration.

The compound or composition may suitably be in liquid form, and may be applied by spraying, drenching or dropping onto the plant. According to some embodiments, the compound or composition is applied by drenching. According to some embodiments, compound or composition is applied by spraying. According to some embodiments, the composition is applied by dropping.

The compound, either as raw material or in the form of a composition, may be applied at any effective amount, for example, at a concentration ranging from about 0.1 µM to about 100 mM. Generally, a effective amount is one at which the active spinosyn compound shows insecticidal activity. The effective amount may thus vary depending on the actual active spinosyn compound employed and can be determined by the skilled person.

The compound or composition may be applied at least once a week. For example, it may be applied 1 to 3 times a week, such as two times a week. The compound may be applied at least once a day. For example, it may be applied 1 to 3 times a day, such as twice a day.

In a further aspect, the present invention provides a process for the preparation of a compound of general formula (I) as defined herein (with R₆ being -H or -OR_{6'}), said process comprising reacting a compound of formula (p-I) 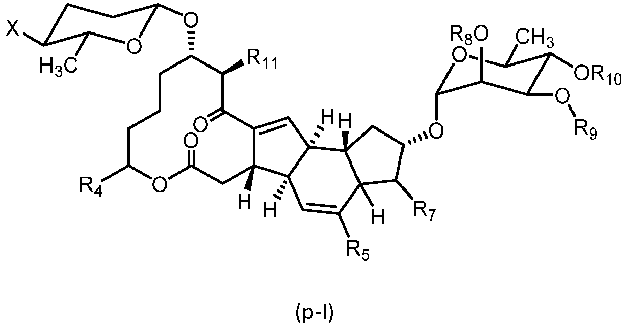 with SeO₂ to obtain a compound according to general formula (I-xi) 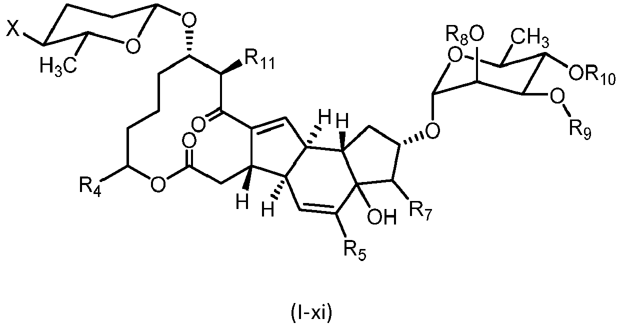 optionally further reacting the resulting compound of general formula (I-xi) with a compound of a general formula R_{6'}-Z to obtain a compound of general formula (I-xii) 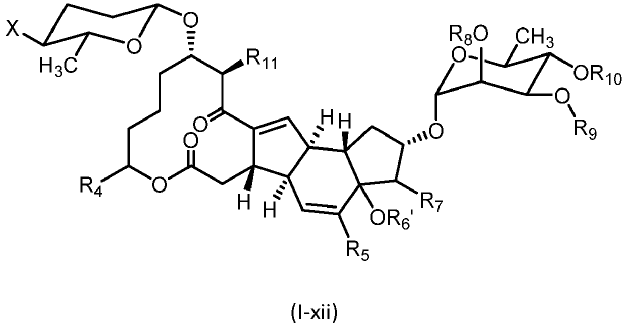 optionally further reacting the compound of general formula (I-xii) with hydrogen (H₂);
wherein Z is a halogen, preferably CI, Br or I, R_{6'} is substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl; and X, R₄, R₅, R₇, R₈, R₉, R₁₀ and R₁₁ are as defined herein.

In a further aspect, the present invention provides a process for the preparation of a compound of general formula (I) as defined herein (with R₆ being -OC(O)H), said process comprising reacting a compound of formula (p-I) 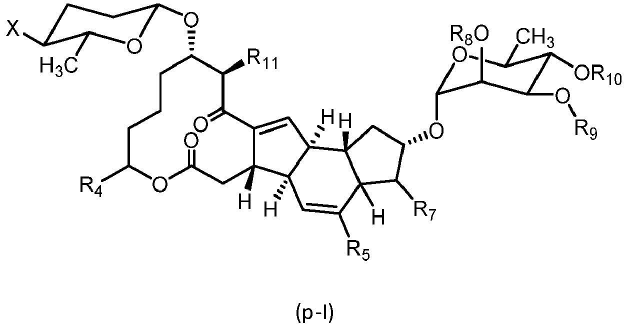 with SeO₂ in the presence of formic acid to obtain a compound according to general formula (I-xxi) 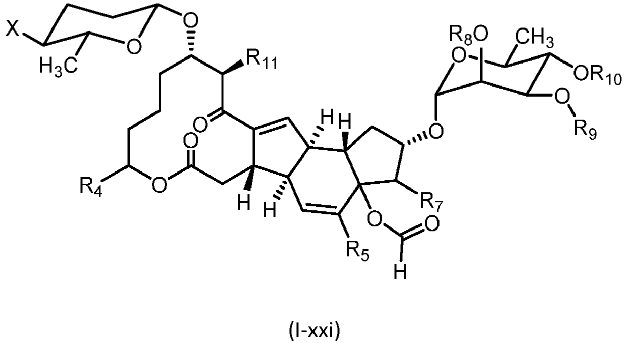 optionally further reacting the compound of general formula (I-xxi) with hydrogen (H₂);
wherein X, R₄, R₅, R₇, R₈, R₉, R₁₀ and R₁₁ are as defined herein.

In a further aspect, the present invention provides a process for the preparation of a compound of general formula (I) as defined herein (with R₆ being -OC(O)R_{6'}), said process comprising reacting a compound of formula (p-I) 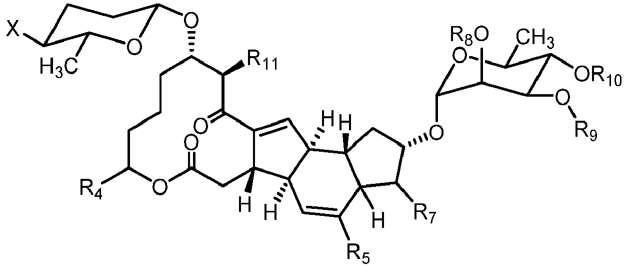 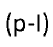 with SeO₂ to obtain a compound according to general formula (I-xi) 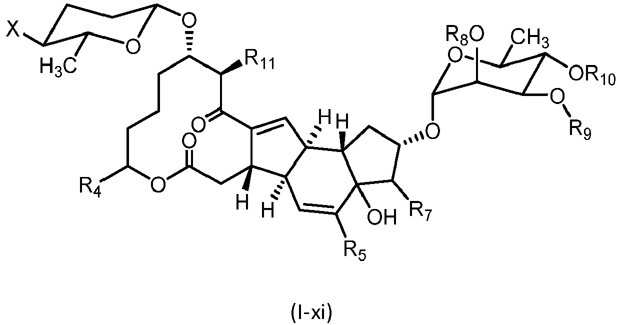 further reacting the resulting compound of general formula (I-xi) with a compound of a general formula R_{6'}C(O)-Z or a compound of a general formula R_{6'}C(O)-O-(O)C- R_{6'} to obtain a compound of general formula (I-xxxi) 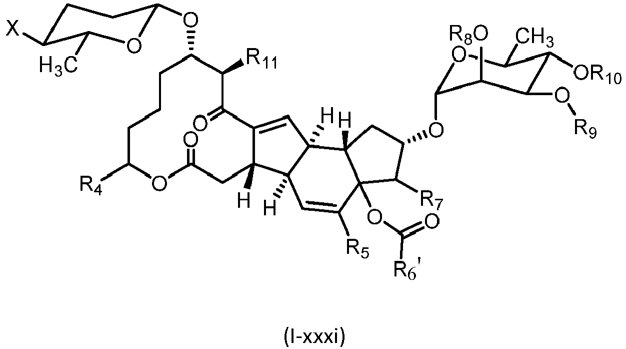 optionally further reacting the compound of general formula (I-xxxi) with hydrogen (H₂);
wherein Z is a halogen, preferably CI, Br or I, R_{6'} is substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and - (C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl; and X, R₄, R₅, R₇, R₈, R₉, R₁₀ and R₁₁ are as defined herein.

If not commercially available, the necessary starting materials for the processes of the invention may be made by procedures which are selected from standard organic chemistry techniques, techniques which are analogous to the synthesis of known structurally similar compounds, or techniques, which are analogous to the procedures described in the examples.

The reaction between a compound of formula (p-I) and SeO₂ is preferably carried out in a suitable solvent, preferably dioxane, at a suitable temperature, such as between room temperature and the reflux temperature, preferably between 90°C and 110°C Generally, the reaction may be carried out for any period of time suitable for the formation of a compound of the invention. Suitably, the reaction is carried out for at least 3 hours, such as from 3 to 12 hours.

Optionally, the reaction between a compound of formula (I-xi) or (I-xxi) and the compound R_{6'}-Z is preferably carried out in a suitable solvent, preferably tetrahydrofuran (THF), preferably in the presence of sodium hydride (NaH), at a suitable temperature, preferably at 20°C Generally, the reaction may be carried out for any period of time suitable for the formation of a compound of the invention. Suitably, the reaction is carried out for at least 3 hours, such as from 3 to 6 hours.

Optionally, a compound of formula (I-xii), (I-xxi) or (I-xxxi) may be reacted with hydrogen in the presence of a suitable catalyst, preferably chloridotris(triphenylphosphine)rhodium(I) (Wilkinson's catalyst), preferably dissolved in a suitable solvent (homogeneous catalysis) to obtain a saturated ring. Generally, the reaction may be carried out for any period of time suitable for the formation of a compound of the invention. Suitably, the reaction is carried out for at least 3 hours, such as from 3 to 42 hours. Generally, the reaction may be carried out at any pressure. Suitably the reaction is carried out at increased pressure of at least 1.1 bar, such as from 1.1 bar to 5 bar, most suitably 3 bar.

Alternatively, the reaction can also be carried out in the presence of the Pearlman's catalyst (moist Pd(OH)2/C) (heterogeneous catalysis) and a suitable solvent, preferably ethanol. Generally, the reaction may be carried out for any period of time suitable for the formation of a compound of the invention. Suitably, the reaction is carried out for at least 1 hour, such as from 1 to 6 hours. Generally, the reaction may be carried out at any pressure. Suitably the reaction is carried out at increased pressure of at least 1.1 bar, such as from 1.1 bar to 5 bar, most suitably 3 bar.

It is to be understood that the methodology described for compounds of general formula (I) applies *mutatis mutandis* to obtain corresponding compounds according to general formula (IIa), (IIb), (IIc), (IIIa), (IIIb), (IIIc), (IIId) and (IIIe).

The process of the present invention may further comprise an purification step after completion of any of the reactions. The purification step may include any any conventional procedure for purification of chemical compounds from a reaction. Well-known purification procedures include centrifugation or filtration, precipitation, and chromatographic methods such as e.g. ion exchange chromatography, gel filtration chromatography, etc.

### Certain definitions

In the context of this invention, a "pesticide" is a compound or composition that is meant to control a plan pests, and includes insecticide, nematicide and molluscicide.

In the context of this invention, a "insecticide" is a compound or composition used for reducing or eliminating insects harmful to cultivated plants.

In the context of this invention, "alkyl" is understood as meaning saturated, linear or branched hydrocarbons, which may be unsubstituted or substituted once or several times. It encompasses e.g. -CH₃ and -CH₂-CH₃. In these radicals, C₁-C₂ alkyl represents C1- or C2-alkyl, C₁-C₃ alkyl represents C1-, C2- or C3-alkyl, C₁-C₄ alkyl represents C1-, C2-, C3- or C4-alkyl, C₁-C₅ alkyl represents C1-, C2-, C3-, C4-, or C5-alkyl, C₁-C₆ alkyl represents C1-, C2-, C3-, C4-, C5- or C6-alkyl, C₁-C₇ -alkyl represents C1-, C2-, C3-, C4-, C5-, C6- or C7-alkyl, C₁-C₈ alkyl represents C1-, C2-, C3-, C4-, C5-, C6-, C7- or C8-alkyl, C₁-C₁₀ alkyl represents C1-, C2-, C3-, C4-, C5-, C6-, C7-, C8-, C9- or C10-alkyl, C₁-C₁₈ -alkyl represents C1-, C2-, C3-, C4-, C5-, C6-, C7-, C8-, C9-, C10-, C11-, C12-, C13-, C14-, C15-, C16-, C17- or C18-alkyl, and C₁-C₂₀ - alkyl represents C1-, C2-, C3-, C4-, C5-, C6-, C7-, C8-, C9-, C10-, C11-, C12-, C13-, C14-, C15-, C16-, C17-, C18-, C19 or C20-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably alkyl is understood in the context of this invention as C₁-C₈ alkyl like methyl, ethyl, propyl, butyl, pentyl, hexyl, heptyl, or octyl; more preferably as C₁-C₆ alkyl like methyl, ethyl, propyl, butyl, pentyl, or hexyl; and most preferably as C₁-C₄ alkyl like methyl, ethyl, propyl or butyl.

"Alkenyl" is understood as meaning unsaturated, linear or branched hydrocarbons, which may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₁-C₁₀ alkenyl or C₁-C₈ alkenyl like ethylene, propylene, butylene, pentylene, hexylene, heptylene or octylene; or is C₁-C₆ alkenyl like ethylene, propylene, butylene, pentylene, or hexylene; or is C₁-C₄ alkenyl, like ethylene, propylene, or butylenes.

"Alkynyl" is understood as meaning unsaturated, linear or branched hydrocarbons, which may be unsubstituted or substituted once or several times. It encompasses groups like e.g. -C=C-CH₃ (1-propinyl). Preferably alkynyl in the context of this invention is C₁-C₁₀ alkynyl or C₂₋₈-alkynyl like ethyne, propyne, butyene, pentyne, hexyne, heptyne, or octyne; or is C₁-C₆ alkynyl like ethyne, propyne, butyene, pentyne, or hexyne; or is C₁-C₄ alkynyl like ethyne, propyne, butyene, pentyne, or hexyne.

In connection with alkyl (also in alkylaryl, alkylheterocyclyl or alkylcycloalkyl), alkenyl and alkynyl - unless defined otherwise - the term "substituted" in the context of this invention is understood as meaning replacement of at least one hydrogen radical on a carbon atom by halogen (F, CI, Br, I), cyano, hydroxy, amino or carboxyl. More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

In the context of this invention "haloalkyl" is understood as meaning an alkyl being substituted once or several times by a halogen (selected from F, CI, Br, I). It encompasses e.g. -CH₂Cl, -CH₂F, -CHCl₂, - CHF₂, -CCl₃, -CF₃ and -CH₂-CHCl₂. Preferably haloalkyl is understood in the context of this invention as halogen-substituted C₁₋₄-alkyl representing halogen substituted C1-, C2-, C3- or C4-alkyl. The halogen-substituted alkyl radicals are thus preferably methyl, ethyl, propyl, and butyl. Preferred examples include -CH₂Cl, -CH₂F, -CHCl₂, -CHF₂, and -CF₃.

In the context of this invention "haloalkoxy" is understood as meaning an -O-alkyl being substituted once or several times by a halogen (selected from F, CI, Br, I). It encompasses e.g. -OCH₂Cl, -OCH₂F, - OCHCl₂, -OCHF₂, -OCCl₃, -OCF₃ and -OCH₂-CHCl₂. Preferably haloalkyl is understood in the context of this invention as halogen-substituted -O-C₁-C₄ alkyl representing halogen substituted C1-, C2-, C3- or C4-alkoxy. The halogen-substituted alkyl radicals are thus preferably O-methyl, O-ethyl, O-propyl, and O-butyl. Preferred examples include -OCH₂Cl, -OCH₂F, -OCHCl₂, -OCHF₂, and -OCF₃.

In the context of this invention "cycloalkyl" is understood as meaning saturated and unsaturated (but not aromatic) cyclic hydrocarbons (without a heteroatom in the ring), which can be unsubstituted or once or several times substituted. Furthermore, C₃-C₁₂ cycloalkyl represents C3-, C4-, C5-, C6-, C7- , C8-, C9-, C10-, C11- or C12-cycloalkyl, C₃-C₄ cycloalkyl represents C3- or C4-cycloalkyl, C₃-C₅ cycloalkyl represents C3-, C4- or C5-cycloalkyl, C₃-C₆ cycloalkyl represents C3-, C4-, C5- or C6-cycloalkyl, C₃-C₇ cycloalkyl represents C3-, C4-, C5-, C6- or C7-cycloalkyl, C₃-C₈ cycloalkyl represents C3-, C4-, C5-, C6-, C7- or C8-cycloalkyl, C₄-C₅ cycloalkyl represents C4- or C5-cycloalkyl, C₄-C₆ cycloalkyl represents C4-, C5- or C6-cycloalkyl, C₄-C₇ cycloalkyl represents C4-, C5-, C6- or C7-cycloalkyl, C₅-C₆ cycloalkyl represents C5- or C6-cycloalkyl and C₅-C₇ cycloalkyl represents C5-, C6- or C7-cycloalkyl. Examples are cyclopropyl, 2-methylcyclopropyl, cyclopropylmethyl, cyclobutyl, cyclopentyl, cyclopentylmethyl, cyclohexyl, cycloheptyl, cyclooctyl, and also adamantly. Preferably in the context of this invention cycloalkyl is C₃-C₈ cycloalkyl like cyclopropyl, cyclobutyl, cyclopentyl, cyclohexyl, cycloheptyl, or cyclooctyl; or is C₃-C₇ cycloalkyl like cyclopropyl, cyclobutyl, cyclopentyl, cyclohexyl, or cycloheptyl; or is C₃-C₆ cycloalkyl like cyclopropyl, cyclobutyl, cyclopentyl or cyclohexyl, especially cyclopentyl or cyclohexyl.

"Aryl" is understood as meaning 3 to 12 membered mono or polycyclic ring systems with at least one aromatic ring but without heteroatoms even in only one of the rings. Examples are phenyl, naphthyl, fluoranthenyl, fluorenyl, tetralinyl or indanyl, 9H-fluorenyl or anthracenyl radicals, which can be unsubstituted or once or several times substituted. Preferably, the aryl is a monocyclic aryl. More preferably the aryl is a 5, 6 or 7 membered monocyclic aryl. Even more preferably the aryl is a 5 or 6 membered monocyclic aryl. Most preferably aryl is understood in the context of this invention as phenyl, naphtyl or anthracenyl, preferably is phenyl.

A "heterocyclyl" radical or group (also called heterocyclyl hereinafter) is understood as meaning 3 to 12 membered mono or polycyclic heterocyclic ring systems, with at least one saturated or unsaturated ring which contains one or more heteroatoms from the group consisting of nitrogen, oxygen and/or sulfur in the ring. A heterocyclic group can also be substituted once or several times.

Examples include non-aromatic heterocyclyls such as tetrahydropyrane, oxazepane, morpholine, piperidine, pyrrolidine as well as heteroaryls such as furan, benzofuran, thiophene, benzothiophene, pyrrole, pyridine, pyrimidine, pyrazine, quinoline, isoquinoline, phthalazine, thiazole, benzothiazole, indole, benzotriazole, carbazole and quinazoline.

Subgroups inside the heterocyclyls as understood herein include heteroaryls and non-aromatic heterocyclyls:
- the heteroaryl (being equivalent to heteroaromatic radicals or aromatic heterocyclyls) is an aromatic 3 to 12 membered mono or polycyclic heterocyclic ring system of one or more rings of which at least one aromatic ring contains one or more heteroatoms from the group consisting of nitrogen, oxygen and/or sulfur in the ring; preferably is an aromatic 3 to 12 membered mono or polycyclic heterocyclic ring system of one or two rings of which at least one aromatic ring contains one or more heteroatoms from the group consisting of nitrogen, oxygen and/or sulfur in the ring, more preferably is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, thiophene and benzimidazole;
- the non-aromatic heterocyclyl is a 3 to 12 membered mono or polycyclic heterocyclic ring system of one or more rings of which at least one ring - with this (or these) ring(s) then not being aromatic - contains one or more heteroatoms from the group consisting of nitrogen, oxygen and/or sulfur in the ring; preferably is a 3 to 12 membered mono or polycyclic heterocyclic ring system of one or two rings of which one or both rings - with this one or two rings then not being aromatic - contain/s one or more heteroatoms from the group consisting of nitrogen, oxygen and/or sulfur in the ring, more preferably is selected from oxazepam, pyrrolidine, piperidine, piperazine, tetrahydropyran, morpholine, indoline, oxopyrrolidine, benzodioxane, oxetane, especially is benzodioxane, morpholine, tetrahydropyran, piperidine, oxopyrrolidine, oxetane and pyrrolidine.

Preferably, the heteroaryl is a monocyclic heteroaryl. More preferably the heteroaryl is a 5, 6 or 7 membered monocyclic heteroaryl. Even more preferably the heteroaryl is a 5 or 6 membered monocyclic heteroaryl.

Preferably, the non-aromatic heterocyclyl is a monocyclic non-aromatic heterocyclyl. More preferably the non-aromatic heterocyclyl is a 4, 5, 6 or 7 membered monocyclic non-aromatic heterocyclyl. Even more preferably the non-aromatic heterocyclyl is a 5 or 6 membered monocyclic non-aromatic heterocyclyl.

Preferably in the context of this invention "heterocyclyl" is defined as a 5 to 10 membered mono or polycyclic heterocyclic ring system of one or two saturated or unsaturated rings of which at least one ring contains one or more heteroatoms from the group consisting of nitrogen, oxygen and/or sulfur in the ring.

Preferred examples of heterocyclyls include oxetane, oxazepan, pyrrolidine, imidazole, oxadiazole, tetrazole, pyridine, pyrimidine, piperidine, piperazine, benzofuran, benzimidazole, indazole, benzodiazole, thiazole, benzothiazole, tetrahydropyrane, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole and quinazoline, especially is pyridine, pyrazine, indazole, benzodioxane, thiazole, benzothiazole, morpholine, tetrahydropyrane, pyrazole, imidazole, piperidine, thiophene, indole, benzimidazole, pyrrolo[2,3b]pyridine, benzoxazole, oxopyrrolidine, pyrimidine, oxazepane, oxetane and pyrrolidine.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

In the context of this invention "alkylaryl" is understood as meaning an aryl group (see above) being connected to another atom through a C₁₋₆-alkyl (see above) which may be branched or linear and is unsubstituted or substituted once or several times. Preferably alkylaryl is understood as meaning an aryl group (see above) being connected to another atom through 1 to 4 (-CH₂-) groups. Most preferably alkylaryl is benzyl (i.e. -CH₂-phenyl).

In the context of this invention "alkylheterocyclyl" is understood as meaning an heterocyclyl group being connected to another atom through a C₁₋₆-alkyl (see above) which may be branched or linear and is unsubstituted or substituted once or several times. Preferably alkylheterocyclyl is understood as meaning an heterocyclyl group (see above) being connected to another atom through 1 to 4 (-CH₂-) groups. Most preferably alkylheterocyclyl is -CH₂-pyridine.

In the context of this invention "alkylcycloalkyl" is understood as meaning an cycloalkyl group being connected to another atom through a C₁₋₆-alkyl (see above) which may be branched or linear and is unsubstituted or substituted once or several times. Preferably alkylcycloalkyl is understood as meaning an cycloalkyl group (see above) being connected to another atom through 1 to 4 (-CH₂-) groups. Most preferably alkylcycloalkyl is -CH₂-cyclopropyl.

Preferably, the cycloalkyl is a monocyclic cycloalkyl. More preferably the cycloalkyl is a 3, 4, 5, 6, 7 or 8 membered monocyclic cycloalkyl. Even more preferably the cycloalkyl is a 3, 4, 5 or 6 membered monocyclic cycloalkyl.

In connection with aryl (including alkyl-aryl), cycloalkyl (including alkyl-cycloalkyl), or heterocyclyl (including alkyl-heterocyclyl), substituted is understood - unless defined otherwise - as meaning substitution of the ring-system of the aryl or alkyl-aryl, cycloalkyl or alkyl-cycloalkyl; heterocyclyl or alkyl-heterocyclyl with one or more of halogen (F, CI, Br, I), cyano, hydroxy, amino, carboxyl, haloalkyl, haloalkoxy, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl-group; a saturated or unsaturated, linear or branched, substituted or unsubstituted -C(O)-O-C₁₋₆-alkyl-group; -NH-Boc and -Boc. Preferably, suitable substituents are 1, 2 or 3 substituents independently selected from fluoro, chloro, bromo, iodo, cyano, nitro, amino, carboxyl, methylamino, dimethylamino, hydroxy, methyl, ethyl, methoxy, methylthio, methylsulfinyl, methylsulfonyl, phenyl, -NH-Boc and -Boc.

Additionally to the above-mentioned substitutions, in connection with cycloalkyl (including alkyl-cycloalkyl), or heterocycly (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl; non-aromatic heterocyclyl or non aromatic alkyl-heterocyclyl with 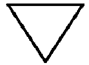 or =O.

A ring system is a system consisting of at least one ring of connected atoms but including also systems in which two or more rings of connected atoms are joined with "joined" meaning that the respective rings are sharing one (like a spiro structure), two or more atoms being a member or members of both joined rings.

The term "salt" is to be understood as meaning any form of the compound according to the invention in which it assumes an ionic form or is charged and is coupled with a counter-ion (a cation or anion), or is in solution. By this are also to be understood complexes of the compound with other molecules and ions, in particular complexes via ionic interactions.

Salts can be formed with anions or acids and in the context of this invention is understood as meaning salts of at least one of the compounds according to the invention with at least one, preferably inorganic, anion. Non-limiting examples of an anion include chloride, sulfate, nitrate, phosphate, citrate, tartrate, acetate, lactate, propionate, gluconate and others.

Salts can also be formed with cations or bases and in the context of this invention is understood as meaning salts of at least one of the compounds used according to the invention - usually a (deprotonated) acid - as an anion with at least one, preferably inorganic, cation. The salts of the alkali metals and alkaline earth metals are particularly preferred, and also those with NH₄, but in particular (mono)- or (di)sodium, (mono)- or (di)potassium, magnesium or calcium salts.

Where a numerical limit or range is stated herein, the endpoints are included. Also, all values and sub ranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example 1: Synthesis of compound A1

A sample of butenyl spinosyn α1 (1.0 g, 1.32 mmol) was dissolved in a mixture of solvents comprise of 20 mL dioxane and 2 mL of water. Selenium dioxide (293 mg, 2.64 mmol) was added and the mixture was heated to 100 °C and stirred for 12 hours. After the reaction was completed the mixture was cooled to room temperature and concentrated by using rotary evaporator. The residue was purified by preparative HPLC on C18 reversed phase column. Chromatographically pure fractions were dried by lyophilization and subjected to LCMS and ¹H NMR analysis. The structure was determined to be 7-hydroxy butenyl spinosyn α1 (compound A1) as a light yellow solid (236 mg, 23.1%).

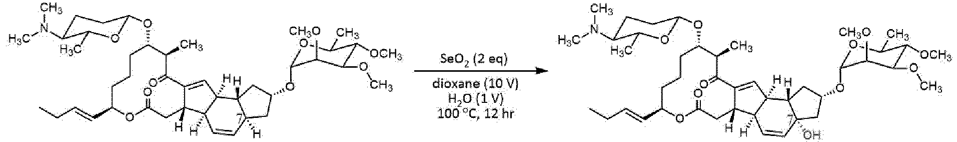

Analytical data for compound A1:
¹H NMR (400 MHz, CDCl₃): δ ppm 6.79 (s, 1H), 6.12 (dd, 1H, J=2.2, 9.9 Hz), 5.93 (dd, 1H, J=3.1, 9.7 Hz), 5.67 (td, 1H, J=6.2, 15.4 Hz), 5.31 (dd, 1H, *J*=7.4, 15.3 Hz), 5.0-5.1 (m, 1H), 4.88 (d, 1H, *J*=0.9 Hz), 4.4-4.5 (m, 2H), 3.6-3.7 (m, 1H), 3.4-3.6 (m, 13H), 3.3-3.4 (m, 1H), 3.1-3.3 (m, 3H), 3.0-3.1 (m, 1H), 2.3-2.4 (m, 1H), 2.25 (s, 8H), 2.0-2.1 (m, 3H), 1.9 (m, 2H), 1.6-1.9 (m, 5H), 1.5-1.6 (m, 6H), 1.4-1.5 (m, 2H), 1.2-1.4 (m, 8H), 1.20 (d, 3H, *J*=6.8 Hz), 0.95 (t, 3H, *J*=7.4 Hz).
LCMS (ESI+): m/z 774.5 [M+H]⁺.

### Example 2: Synthesis of compound H1

A sample of 7-hydroxylated butenyl spinosyn α1 (250 mg, 323 µmol) was dissolved in 6.25 mL of tetrahydrofuran and sodium hydride (19.4 mg, 484 µmol, 60% purity) was added. After 10 minutes of mixing iodomethane (68.8 mg, 484 µmol) was added and stirred at 20 °C for 5 hours. The reaction solution was quenched with water and purified by preparative HPLC to obtain pure 7-methoxy butenyl spinosyn α1 (63.1 mg, 24.8% yield, 99.7% purity) as a white solid. The structure of the compound was determined using LCMS and ¹H NMR.

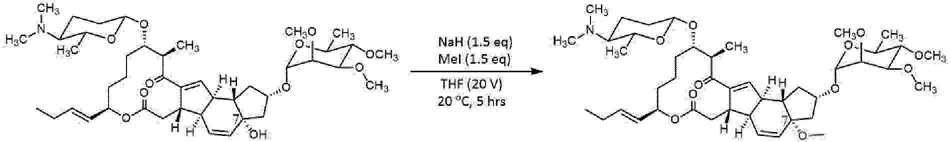

Analytical data for compound H1:
¹H NMR (400 MHz, CDCl₃): δ ppm 6.77 (br s, 1H), 6.04 (dd, J=2.4, 9.8 Hz, 1H), 5.92 (dd, J=2.8, 9.8 Hz, 1H), 5.68 (td, J=6.3, 15.3 Hz, 1H), 5.30 (dd, J=7.3, 15.4 Hz, 1H), 5.12 - 4.99 (m, 1H), 4.85 (s, 1H), 4.44 (br d, J=7.5 Hz, 1H), 4.31 (q, J=6.9 Hz, 1H), 3.71 - 3.46 (m, 16H), 3.39 - 3.28 (m, 2H), 3.21 - 3.08 (m, 5H), 3.04 (br s, 1H), 2.54 - 2.19 (m, 8H), 2.18 - 2.07 (m, 2H), 2.06 -1.87 (m, 3H), 1.79 (dt, J=6.5, 12.7 Hz, 3H), 1.72 - 1.43 (m, 9H), 1.38 - 1.13 (m, 11H), 0.95 (t, J=7.4 Hz, 3H).
LCMS (ESI+): m/z 788.6 [M+H]⁺.

### Example 3: Synthesis of compound H2

A sample of 7-hydroxylated butenyl spinosyn α1 (300 mg, 387.6 µmol) was dissolved in 6 mL of dimethylformamide and sodium hydride (23.3 mg, 581.4 µmol, 60% purity) was added. After 10 minutes of mixing ethyl iodide (90.7 mg, 581.4 µmol) was added and stirred at 20 °C for 3 hours. The reaction mixture was further purified by preparative HPLC to obtain pure 7-ethoxy butenyl spinosyn α1 (86.1 mg, 27.7% yield, 99.6% purity) as a white solid. The structure of the compound was determined using LCMS and ¹H NMR.

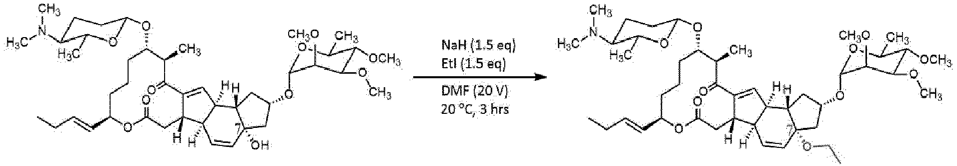

Analytical data for compound H2:
¹H NMR (400 MHz, CDCl₃): δ ppm 6.78 (br s, 1H), 6.10 - 5.98 (m, 1H), 5.89 (br dd, J=2.8, 9.8 Hz, 1H), 5.75 - 5.62 (m, 1H), 5.31 (br dd, J=7.1, 15.4 Hz, 1H), 5.11 - 5.00 (m, 1H), 4.85 (s, 1H), 4.43 (br d, J=7.1 Hz, 1H), 4.35 - 4.22 (m, 1H), 3.76 - 3.43 (m, 16H), 3.43 - 3.24 (m, 4H), 3.19 - 3.09 (m, 2H), 3.04 (br s, 1H), 2.50 - 2.33 (m, 1H), 2.24 (s, 7H), 2.17 - 2.06 (m, 3H), 2.05 - 1.94 (m, 3H), 1.91 - 1.72 (m, 4H), 1.69 - 1.41 (m, 7H), 1.28 (br dd, J=6.2, 9.9 Hz, 7H), 1.19 (br d, J=6.6 Hz, 3H), 1.09 (br t, J=6.9 Hz, 3H), 1.02 - 0.89 (m, 3H).
LCMS (ESI+): m/z 802.5 [M+H]⁺.

### Example 4: Evaluation of kinetic solubility of spinosyn compounds

Solubility of the compounds of the invention was assessed using kinetic solubility assay and compared to solubility of butenyl spinosyn. Stock solutions of spinosyn compounds were prepared in DMSO at 10 mM concentration). 10 uL of the stock solutions were added into 490 of in K.S. 5.4 buffer of pH 5.4 (80mM phosphoric acid, acetic acid, boric acid buffer, adjusted the pH to pH 5.4 with 1 N HCI and IN NaOH) or K.S. 7.4 buffer with pH of 7.4 (80mM phosphoric acid, acetic acid, boric acid buffer, adjusted the pH to pH 7.4 with 1 N HCI and IN NaOH). The solutions were incubated with shaking at room temperature (25 ±2 °C) for 24 hours. 200 uL of each solution were then transferred into a new MultiScreen filter plate (Membrane of polycarbonate) and filtered by millipore vacuum manifold, and the filtrate was collected. The concentrations of tested compounds in the filtered samples were analyzed by HPLC analysis calibrated by a standard curve generated by injection of 3 standard solutions of the relevant compound with concentrations of 1, 20, 200 µM. The results of the testing are presented in Table 1. The conditions of HPLC analysis are presented in Table 2.

**Table 1. Comparison of solubility of spinosyn compounds at pH 5.4 and 7.4.**

| **Compound** | **K.S 5.4 Dose Conc (µM)** | **Kinetic Solubility pH=5.4 (µM)** | **K.S 7.4 Dose Conc (µM)** | **Kinetic Solubility pH=7.4 (µM)** |
|---|---|---|---|---|
| **Butenyl spinosyn** | 200 | 183.17 | 200 | 3.52 |
| **A1** | 200 | 192.40 | 200 | 186.57 |
| **H1** | 200 | 182.65 | 200 | 77.12 |
| **H2** | 200 | 166.38 | 200 | 7.68 |

**Table 2. Conditions of the HPLC analysis for determination of kinetic solubility.**

| | | | |
|---|---|---|---|
| Instrument | Agilent 1200 | | |
| Detector | DAD | | |
| Mobile phase | A: Water containing 0.37‰ TFA | | |
| | B: Acetonitrile containing 0.19‰ TFA | | |
| Gradient | Time | B | Flow rate |
| | (min) | (%) | (mL/min) |
| | 0.00 | 5 | 1.0 |
| | 2.00 | 90 | 1.0 |
| | 2.50 | 90 | 1.0 |
| | 3.01 | 5 | 1.0 |
| | 4.00 | 5 | 1.0 |
| Column | Xbridge C18 (2.1×50 mm, 5 µm) | | |
| Injection Volume | 20 µL | | |

These results show that the compounds of the invention are significantly more soluble in the neutral pH values (7.4) compared to butenyl spinosyn, which represents a big advantage in preparation of formulations based on aqueous media.

### Example 5 Evaluation of biological activity of spinosyn compounds

Insecticide activities of the compounds of this invention were assessed in comparison with commercially available samples of spinosad (LGC (Dr. Ehrenstorfer); Product code: DRE-C16972830) and spinetoram (LGC (Dr. Ehrenstorfer); Product code: DRE-C16972770) Solutions of spinosyn compounds were prepared by adding 0.05% Tween 80, lowering pH to 5.95 and sonicating. Two concentrations of each compound were used, 0.1 µg/mL and 0.3 µg/mL, with each condition tested in four parallels.

Freshly collected corn leaves were dipped into the prepared solutions. After dipping leaves were left to dry at room temperature for 1 hour. Each dry leaf was then infested with 4 larvae (development stage L1), and the experimental device was incubated at 21 °C - 23°C for 24 h in order to optimize insect development. At two timings post infestation (3 days and 6 days), the insecticide efficacy is evaluated from the number of living larvae using the Henderson-Tilton method. Untreated control was integrated for normalization and validation of larvae health.

**Table 3: Comparison of efficacy of tested spinosyn compounds**

| **Treatment** | **Dose (µg a.i./ml)** | **% Efficacy** | |
|---|---|---|---|
| | | **3 days** | **6 days** |
| SPN | 0.1 | 25% | 46% |
| | 0.3 | 63% | 100% |
| Spinoteram | 0.1 | 58% | 81% |
| | 0.3 | 85% | 96% |
| Butenyl spinosyn | 0.1 | 33% | 56% |
| | 0.3 | 79% | 100% |
| A1 | 0.1 | 2% | 2% |
| | 0.3 | 15% | 19% |
| H1 | 0.1 | 27% | 89% |
| | 0.3 | 58% | 100% |

These results show that the compound H1 shows extremely potent insecticidal activity on *Ostrinia nubilialis* larvae. The activity is comparable or superior to Spinosad and spinetoram.

### Example 6: UV-stability of spinosyn compounds

Synthesized spinosyn compounds were used to prepare 5 mM solutions in acetonitrile. The solution was divided in 2 aliquots and one aliquot was exposed to UV light at 254 nm and the other aliquot was incubated in the same conditions without UV light exposure. Quantities of remaining starting compounds were determined by HPLC after 0h, 1h, 5h and 10 h. The results are presented in Table 4.

**Table 4. shares of remaining compounds after exposure to UV light.**

| Compound/treatment | 0 h | 1 h | 5h | 10 h | Purity changing |
|---|---|---|---|---|---|
| **Butenyl spinosyn (1) under UV** | 90.0% | 88.6% | 70.9% | 59.6% | -30.4% |
| **Butenyl spinosyn (1) without UV** | | 90.6% | 90.7% | 89.4% | -0.6% |
| **A1 under UV** | 90.7% | 86.6% | 71.3% | 52.2% | -38.5% |
| **A1 without UV** | | 90.7% | 90.8% | 90.9% | +0.2% |
| **H1 under UV** | 96.0% | 95.6% | 87.5% | 73.5% | -22.5% |
| **H1 without UV** | | 96.5% | 96.9% | 96.7% | +0.7% |
| **H2 under UV** | 93.2% | 87.6% | 67.8% | 39.7% | -53.5% |
| **H2 without UV** | | 93.6% | 93.7% | 92.3% | -0.9% |

### List of certain references cited in the description

Donald R. Hahn, Gary Gustafson, Clive Waldron, Brian Bullard, James D. Jackson and Jon Mitchell; "Butenyl-spinosyns, a natural example of genetic engineering of antibiotic biosynthetic genes"; J Ind Microbiol Biotechnol.; 33(2):94-104 (2006) https://doi.org/10.1007/s10295-005-0016-9
Chao Guo, Weiqun Guo, Yuchun Liu and Chao Wang; "Complete genome sequence of butenyl-spinosyn-producing Saccharopolyspora strain ASAGF58"; Annals of Microbiology; 70, 46 (2020) https://doi.org/10.1186/s13213-020-01587-4
Paul Lewer, Donald R. Hahn, Laura L. Karr, Dennis O. Duebelbeis, Jeffrey R. Gilbert, Gary D. Crouse, Thomas Worden, Thomas C. Sparks, Pat McKamey, Rex Edwards abd Paul R. Graupner; "Discovery of the butenyl-spinosyn insecticides: novel macrolides from the new bacterial strain Saccharopolyspora pogona"; Bioorg Med Chem.; 17(12):4185-96 (2009) https://doi.org/10.1016/j.bmc.2009.02.035
John Daeuble, Thomas C. Sparks, Peter Johnson and Paul R. Graupner; "Modification of the butenyl-spinosyns utilizing cross-metathesis"; Bioorg Med Chem.; 17(12):4197-205 (2009) https://doi.org/10.1016/j.bmc.2009.02.036Get
Herbert A Kirst; "The spinosyn family of insecticides: realizing the potential of natural products research"; J Antibiot; 63:101-111 (2010) https://doi.org/10.1038/ja.2010.5
Carl V. DeAmicis, James E. Dripps, Chris J. Hatton, and Laura L. Karr; "Physical and Biological Properties of the Spinosyns: Novel Macrolide Pest-Control Agents from Fermentation"; ACS Symposium Series; Vol. 658, Chapter 11: 144-154 (1997) https://doi.org/10.1021/bk-1997-0658.ch011
Da-You Ma, Long-Long Wang, Qin Lai, Kun-Jian Peng, Xuan Li, Zeng-Xia Li, Li-Jun Liu, Zhi-Yong Luo and Su-You Liu; "Synthesis and antiproliferative activities of novel quartenary ammonium spinosyn derivatives"; Bioorg Med Chem Lett.; 28(20):3346-3349 (2018) https://doi.org/10.1016/j.bmcl.2018.09.005
Raghavendra Ramachanderan and Bernd Schaefer; "Spinosyn insecticides"; ChemTexts; 6, 20 (2020) https://doi.org/10.1007/s40828-020-00113-y

## Claims

1. A compound of general formula (I) 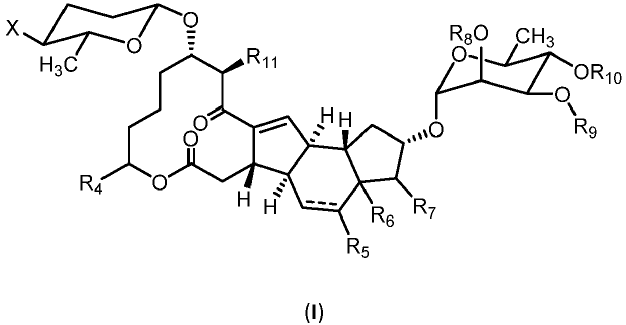 wherein
the dashed line is a single bond, a double bond or an epoxide;
X is -NR₂R₃, -NHR₂, -N⁺R₁R₂R₃ or -OR₂;
R₁ is -(CH₂)n-O-C(O)-R₁b, wherein
n is an integer ranging from 0 to 20; preferably is an integer ranging from 1 to 10, more preferably is an integer ranging from 1 to 5; and
Rib is selected from the group consisting of -C(R₂b)₃, -N(R₂b)₂, -OH and -OC(R₂b)₃, wherein
each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₂ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
R₃ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; more preferably is unsubstituted methyl;
R₄ is selected from the group consisting of unsubsituted butyl, unsubsituted 1-butenyl, unsubsituted 1,3-butadienyl and unsubsituted 3-hydroxy-1-butenyl, preferably is unsubsituted 1-butenyl;
R₅ is -H;
R₆ is -OR_{6'} or -OC(O)R_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted, preferably is substituted or unsubsituted C₁-C₂ alkyl; more preferably is unsubstituted C₁-C₂ alkyl; most preferably is unsubstituted methyl;
R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably R₇ is -H;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl;
optionally in the form of a corresponding salt thereof.

2. The compound according to claim 1, wherein X is -NR₂R₃.

3. The compound according to claim 1 or 2, wherein R₂ is hydrogen or substituted or unsubsituted C₁-C₆ alkyl; and/or R₃ is hydrogen or substituted or unsubsituted C₁-C₆ alkyl.

4. The compound according to any one of claims 1 to 3, wherein R₄ is unsubstituted 1-butenyl.

5. The compound according to any one of claims 1 to 4, wherein R₆ is -OR_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted; preferably is substituted or unsubsituted C₁-C₂ alkyl.

6. The compound according to any one of claims 1 to 4, wherein R₆ is -OC(O)R_{6'}, wherein R_{6'} is hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₆ cycloalkyl, substituted or unsubsituted C₃-C₆ aryl, substituted or unsubsituted C₃-C₁₂ heterocyclyl, -(C₁-C₄ alkyl)-(C₃-C₆ cycloalkyl) with the cycloalkyl being optionally substituted, -(C₁-C₄ alkyl)-(C₃-C₆ aryl) with the aryl being optionally substituted, and -(C₁-C₄ alkyl)-(C₃-C₁₂ heterocyclyl) with the heterocyclyl being optionally substituted; preferably is substituted or unsubsituted C₁-C₂ alkyl.

7. The compound according to any one of claims 1 to 6, wherein R_{6'} is unsubstituted C₁-C₂ alkyl.

8. The compound according to any one of claims 1 to 7, wherein R₇ is -H.

9. The compound according to any one of claims 1 to 8, wherein each of Rg, R₉, R₁₀ and R₁₁ is - CH₃.

10. The compound according to claim 1, which is selected from the group consisting of 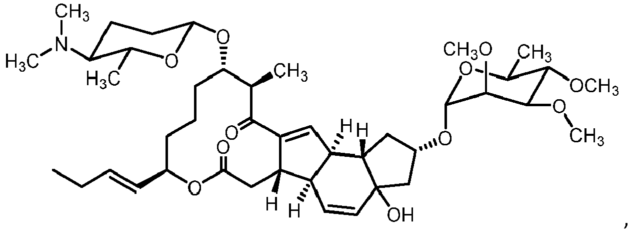 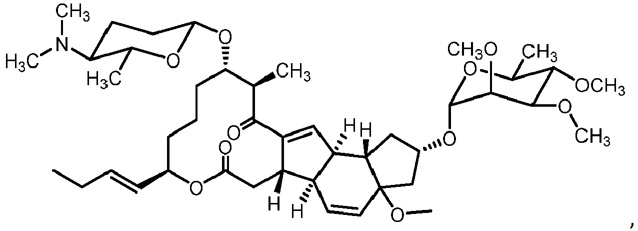 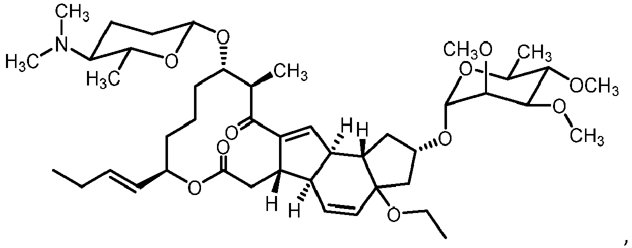 and corresponding salts thereof.

11. Composition comprising a compound according to any one of claims 1 to 10, optionally in the form of a corresponding salt thereof, and one or more physiologically acceptable adjuvants.

12. Use of a compound according to any one of claims 1 to 10, optionally in the form of a corresponding salt thereof, or a composition according to claim 11 as a pesticide.

13. A method for controlling a pest, such as a plant pest, comprises contacting a pest, such as a plant pest, with a compound according to any one of claims 1 to 10, optionally in the form of a corresponding salt thereof, or a composition according to claim 11.

14. A method for protecting a plant against a plant pest, comprising the step of: applying a compound according to any one of claims 1 to 10, optionally in the form of a corresponding salt thereof, or a composition according to claim 11 to a plant in need thereof.

15. The method according to claim 13 or 14, wherein the plant pest is an insect or mite.
